Europäisches Patentamt

European Patent Office

Office européen des brevets

(19)

(11) Publication number : **0 496 174 A1**

## (12) EUROPEAN PATENT APPLICATION

(21) Application number : 91402887.3

(22) Date of filing : 28.10.91

(51) Int. Cl.[5] : **C12N 15/00, C12N 15/85,**
**C12N 15/12, A07K 67/027**

(30) Priority : **29.10.90 US 604905**

(43) Date of publication of application :
**29.07.92 Bulletin 92/31**

(84) Designated Contracting States :
**AT BE CH DE DK ES FR GB GR IT LI LU NL SE**

(71) Applicant : **INSTITUT PASTEUR**
**25-28, rue du Docteur Roux**
**F-75724 Paris Cédex 15 (FR)**
(71) Applicant : **INSTITUT NATIONAL DE LA**
**SANTE ET DE LA RECHERCHE MEDICALE**
**(INSERM)**
**101, rue de Tolbiac**
**F-75654 Paris Cédex 13 (FR)**

(72) Inventor : **Pringault, Eric**
**30, rue du Docteur Roux**
**F-75015 Paris (FR)**
Inventor : **Robine, Sylvie**
**3, rue R. Marcheron**
**F-92170 Vanves (FR)**
Inventor : **Huet, Christian**
**35, rue M. Regnier**
**F-75015 Paris (FR)**
Inventor : **Babinet, Charles**
**158, Bd. Vincent-Auriol**
**F-75013 Paris (FR)**
Inventor : **Louvard, Charles**
**23, allée de Trevise**
**F-92330 Sceaux (FR)**

(74) Representative : **Desaix, Anne et al**
**Ernest Gutmann - Yves Plasseraud S.A., 67**
**bld.Haussmann**
**F-75008 Paris (FR)**

(54) **Villin gene promoter sequence and its use in vectors, transformed mammalian cell lines, transgenic animals, and cell lines derived from the animals.**

(57)    An isolated DNA sequence encoding a promoter of the human villin gene is provided. The DNA sequence can be operatively linked to a nucleotide sequence of a gene, such as an oncogene, and incorporated in cloning and expression vectors. The vectors are useful in the production of transgenic, non-human mammals as models of colorectal cancer.

EP 0 496 174 A1

Technical Field

This invention relates to a gene promoter sequence for regulating gene expression in mammalian cells. This invention also relates to vectors containing the promoter sequence, cells transformed with the vectors, transgenic animals based on the vectors, and cell lines derived from cells in the animals.

Background Art

The ability to introduce genes into the germ line of mammals is of great interest in biology. The propensity of mammalian cells to take up exogenously added DNA and to express genes included in the DNA has been known for many years. The results of gene manipulation are inherited by the offspring of these animals. All cells of these offspring inherit the introduced gene as part of their genetic make-up. Such animals are said to be transgenic.

Transgenic mammals have provided a means for studying gene regulation during embryogenesis and in differentiation, for studying the action of oncogenes, and for studying the intricate interaction of cells in the immune system. The whole animal is the ultimate assay system for manipulated genes, which direct complex biological processes.

Transgenic animals can provide a general assay for functionally dissecting DNA sequences responsible for tissue specific or developmental regulation of a variety of genes. In addition, transgenic animals provide useful vehicles for expressing recombinant proteins and for generating precise animal models of human genetic disorders.

By means of recombinant techniques, it is possible to make constructs containing oncogenes. Many oncogene constructs have been introduced into transgenic mice and have been found to elicit tumorigenic responses. For example, mice that carry the SV40 enhancer and region coding for the large T-antigen reproducibly develop tumors of the choroid plexus, which are derived from the cells lining the ventricles of the brain. Palmiter et al., Nature **316**:457-60 (1985). In similar experiments, the *c-myc* oncogene was fused to the LTR of mouse mammary tumor virus. In one transgenic line, females characteristically developed mammary carcinomas during the second or third pregnancy. Stewart et al., Cell **38**:627-37 (1984). In other experiments, the *c-myc* oncogene was driven by immunoglobulin enhancers and gave rise to malignant lymphoid tumors in transgenic mice. Adams et al., Nature **318**:533-8 (1985). When an oncogenic human *ras* gene was fused to an elastase promoter/enhancer construct, transgenic mice were born with pancreatic neoplasms that appeared to be due to transformation of all of the differentiating pancreatic cells. Palmiter et al., Ann. Rev. Genet. **20**:465-99 (1986).

For a general discussion of gene cloning and expression in animals and animal cells, see Old and Primrose, "Principles of Gene Manipulation," Blackwell Scientific Publications, London (1989), page 255 *et seq.*

Transgenic lines, which have a predisposition to specific tumors, are of great value in the investigation of the events leading to malignant transformation. It is well known that the efficacy of an anti-cancer treatment is dependent on identification of the tumor cells that are the primary cause of the disease. This is especially true in the detection of proliferation of tumor cells of the gastrointestinal tract or of renal origin. Indeed, cancers of the digestive tract are widespread, and there is a critical need for effective treatment for these diseases. The discovery of the effective treatments can be expedited by providing an animal model that will lead to carcinogenesis, which will enable the study of the efficacy, safety, and mode of action of anti-tumoral pharmaceutical agents.

In summary, there exists a need in the art for reagents and methods for providing transgenic animal models of human tumor growth and proliferation, including transgenic animal models of human tumors of the digestive tract and renal system.

Disclosure of the Invention

Accordingly, this invention aids in fulfilling these needs in the art. Specifically, this invention provides an isolated DNA sequence encoding or corresponding to a promoter of the human villin gene. The nucleotide sequence consists essentially of :

PRV.STRIDER -> Restriction Map

DNA   sequence    1991 b.p.    GTCGACCTGCAG ... CTGCAGGTCGAC    linear

```
          Pst I                 Taq I
        BspM I                Sau3A I
      Taq I                   Mbo I
     Sal I                    Dpn I           Mse I                    Alu I
     HinC II        HinC II   Cla I     Hpa I            HinC II  Dde I
     Acc I    BspM I     Alw I           HinC II  Dde I      Mnl I          Mbo II      Acc I
     | |   |  |  |     |    | |     | |       | |     |     |      | |. |        |          |
     GTCGACCTGCAGGTCAACGGATCATCGATTTAGTTAACCCCCTAAGTTGACCCCCTCAGCTCATAGATGAAGAAGGGAAG   80
     CAGCTGGACGTCCAGTTGCCTAGTAGCTAAATCAATTGGGGGGATTCAACTGGGGGAGTCGAGTATCTACTTCTTCCCTTC
     | |   |  |  | •  |     | |     | |    •  | |     • |     |    •   | |   | •      | •           |
     1            9         19            33          42        54             69          80
     1               13            24      33              46        55
     1                        20            34                          58
       2                      20
         5                    20
            7                           25
```

EP 0 496 174 A1

```
                                                        Msp I
                                                        Hpa II
                                                        ScrF I
                                                        Ncl I
                                                        Bcn I
                                                       Sau96 1
                                                       Hae III
                                                       Sau96 I
                                                       Nla IV
                                                      Bsp1286 I
                                                      Ban II   BstU I
                                                      Apa I    HinP I
                                    Mae I             Nla IV   Hha I
            Mae II          Alu I             Mnl I   EcoO109 I
              I               I I               I     III  II   II
TCTACGTTCATACAGCTAGTGAGTGTCAAAGAAAAAGAGGGACGGGGCCCGGCGCGGTGGCTCACGCCTGTAATTCCAGC   160
AGATGCAAGTATGTCGATCACTCACAGTTTCTTTTTCTCCCTGCCCCGGGCCGCGCCACCGAGTGCGGACATTAAGGTCG
      I           •         I I          •              I     •   III  II•  II          •             •            •
     84               94                  117    124
              96                                 124        132
                                                 125        132
                                                 125          133
                                                 125
                                                 125
                                                 125
                                                  126
                                                  126
                                                    128
                                                    128
                                                    128
                                                      129
                                                      129
```

EP 0 496 174 A1

```
                                                              NspH I
                                                             PflM I
                                                             Hae III
                                 Bsu36 I                      Hae I
                                  Hph I                       Eae I
                                 Sau3A I                      Bal I
              Mnl I              Mbo I                        ScrF I   Nsp7524 I
             Dde I              Dpn I      Mnl I              EcoR II  Nla III
           Mnl I               Alw I  Dde I        Taq I     BstN I   Afl III
             |     | |           | | |    | | |               |     | | |  |   | |
AATTTAGGAGGCTGAGGCGGGTGGATCACCTGAGGTCAGGAGTTCGAGACCAGCCTGGCCAACATGTGGTGGAAATCCCG  240
TTAAATCCTCCGACTCCGCCCACCTAGTGGACTCCAGTCCTCAAGCTCTGGTCGGACCGGTTGTACACCACCTTTAGGGC
             | • | |      •    | | |   | | |       •      |       •   | | |  |• | |       •         •
            168          183        190         204      214      222
               172          184        192               214         223
                  174          184                       214         222
                       184                                216
                          186                             216
                             189                          216
                                                          217
                                                            219
                                                              222


                                         HinP I                        Mnl I
                                         BstU I                        Dde I
              Mse I              Alu I      Mnl I  Hha I      Alu I       Mnl I        Hinf I
              |                  |          |      | |        |          |    | |       |
TCTCTATTAAAAATACAAAATTAGCTGGGCTTGAGGCTCGCGCCTGTAATCCAGCTACTTCAGGGAGGCTGAGGCAGGAG  320
AGAGATAATTTTTATGTTTTAATCGACCCGAACTCCGAGCGCGGACATTAGGTCGATGAAGTCCCTCCGACTCCGTCCTC
              | •          •   |       •  |        | |       •   |        •   |    |•|       |
              247              263        273      280        293        305        320
```

EP 0 496 174 A1

```
                                            279                          309
                                              280                          311

                   Nla IV
                  ScrF I
                  Nci I
                  Msp I
                  Hpa II
                  Bcn I
                 Xma I
                 Sma I
                 Sec I
                 ScrF I                            Sec I
                 Nci I                             ScrF I
                 Bcn I        Mnl I                EcoR II
                 Ava I       Sec I         Rsa I   BstN ·I
                 | |    |    | |           |       |
AATCACTTGAACCCGGGAGCCGAGGTTGAAGTAAGCCGAGATTGTGCCATTGTACTTCAGCCTGGGCAACAAGAGTGAAA 400
TTAGTGAACTTGGGCCCTCGGCTCCAACTTCATTCGGCTCTAACACGGTAACATGAAGTCGGACCCGTTGTTCTCACTTT
   •  | |   |    | |         •             •      • |      •|      •              •
     332      340                                372    381
     332        342                                     381
     332                                                381
     332                                                381
     332
     332
     332
      333
      333
      333
      333
      333
       336
```

EP 0 496 174 A1

```
                                                    Hph  I
                                                     |
CTCTGTCCCAAAAAATAAATAAATAAATAAATAAATAAAAGAAGGGGACAGAGACAAAGGTGATGACAGAAATGAAACAGA 480
GAGACAGGGTTTTTATTTATTTATTTATTTATTTATTTTCTTCCCCTGTCTCTGTTTCCACTACTGTCTTTACTTTGTCT
        •           •           •           •           •   |    •           •           •
                                                             458

                                                          Sec  I
                                                        Bsp1286 I
                                          Hinf I   Ban II
                                        ScrF I  Sec I      ScrF I
                                        EcoR II        Nla IV
                  ScrF I               BstN I  ScrF I      EcoR II
                  EcoR II     Sau96 I  EcoR II  BstN I
                  BstN I      Ava II            BstN I  Sec I
                    |            |    |           |      ||    ||
AAACGGTCTCCTACTTTATACCCCAACCTGGTTCTGCTGGACCAGGAATCCTGGGAGCCCTGGGGACTTGGTAGGTTGAA 560
TTTGCCAGAGGATGAAATATGGGGTTGGACCAAGACGACCTGGTCCTTAGGACCCTCGGGACCCCTGAACCATCCAACTT
        •           •       |  •      |• |     |      |     ||    ||•        •           •
                           507       519      530      538
                           507       519      530      539
                           507          522   530      539
                                        522            534
                                        522   530      539
                                            526     535
                                                   535
                                                      539
```

```
                                 Nla IV                                              Sau96 I
                               Nla III                                              Nla IV
                               Sty I                                                Ava II
                 Mnl I        Sec I                      Hae III        Hae III      PpuM I          Sty I
        EcoN I                Nco I                      Hae I          Hae I        EcoO109 I       Sec I
        |              |       I I   I                    I I            I I          I I              I
        AAATACCTGATGGAGGAGACCATGGTTCCTTGATGGAGAAGGCCATAGCCAACAGGCCATCAGACAGGACCCAAGCCCCA 640
        TTTATGGACTACCTCCTCTGGTACCAAGGAACTACCTCTTCCGGTATCGGTTGTCCGGTAGTCTGTCCTGGGTTCGGGGT
        |       •      |       I I   I          •          I I            •   I I      •   I I  •        I  •
        566                   580                      600                614        626              638
                    573        580                      601                615        626              638
                               580                                                   627
                              581                                                   627
                                584                                                 627

                                           Fnu4H I                       AlwN I
                                           Bbv I                         Sau3A I
         Nla IV                          Fnu4H I                       Mbo I
        Bsp1286 I                        Bbv I                         Dpn I
        Ban II           Mae III        Alu I                          Bcl I                          Mnl I
         I I              |              I I   I•                       I I   I  •                      I  •
        AGGGGCTCCTACAGTTACACGGCAAGCTGCTGCTAATCAGTCGGAGCAGGCAATGATCAGAGACTGGGCAGGGCTTACCT 720
        TCCCCGAGGATGTCAATGTGCCGTTCGACGACGATTAGTCAGCCTCGTCCGTTACTAGTCTCTGACCCGTCCCGAATGGA
         I I     •.     |       •      I I   I•              •             •   I I   I  •        •           I  •
        643            654        665                                     694                          718
        643                       666                                     695
         644                      666                                     695
                                   669                                    695
```

8

669

699

Mae I
Xba I

Sec I
ScrF I
EcoR II
BstN I
Bbv I

Fnu4H I
Fnu4H I
Bbv I

Dde I
Mnl I
Dde I
Bsu36 I

Dde I

Sca I

Rsa I

Hae III
Hae I
Mae III

Ple I
Hinf I
Mae III

Hph I
Mnl I
Mae I
Mae III

Dde I
Mae III
Dra III
Mnl I
Hae I

Hae III
Stu I
Hae I
Mnl I

CCGAACTCTAGAACGCCAGGGAAGCAGCAGCCACCCCCTCAGGCTCAGAAAAAGGGAGTTTCTAAGTACTAAAAAGGCCAAGT
GGCTTGAGATCTTGCGGTCCCTTCGTCGTCGGTGGGGGGAGTCCGAGTCTTTTTCCCTCAAAGATTCATGATTTTTCCGGTTCA

727
728
736
736
736
736
736
744
744
747
747
756
756
757
763
780
783
784
792
793
799
800

CACTAAGAGGTGACTCAAGTCAAGGTGCAAGGAGAGATTAGATAGTGAGATTTCAGGCTGTCACGGAGTGGAGGCCTGA
GTGATTCTCCACTGAGTTCCACGTTCCTCTCTAATCTATCACTCTAAAGTCCGACAGTGCCTCACCTCCGGACT

803
807
809
810
812
812
861
863
872
873
873
874
880

EP 0 496 174 A1

```
                                                                      Xho I
          .. Mnl I                               Fnu4H I       Mnl I
      Nla III                                    Bbv I       PaeR7 I
      NspH I                              Nla III   Alu I    Taq I
      Nsp7524 I                   Bsm I      Mnl I          Ava I        Hph I
      | |        |                |       |    |   | |      | | |        |              .
CCTGTCGGCACATGCCCTCTCATTCATTATTCATCAGTTCAAGGAGCATTCATGGAGGGCAGCTCTCTCGAGGCTGGGTG  960
GGACAGCCGTGTACGGGAGAGTAAGTAATAAGTAGTCAAGTTCCTCGTAAGTACCTCCCGTCGAGAGAGCTCCGACCCAC
      | |        |      .        .          .        |    • |    |    | • |    | | |       | •
      890                                          926      935        947        957
      890                                            931        941        948
      891                                                     939      947
          896                                                 939         950
                                                              947


      Nla III
      Sty I
      Sec I    Sau3A I     Mnl I            Nla IV
      Nco I    Mbo I       Hae III    Mnl I      Mnl I        Mnl I              Mae I
  Nla IV       Dpn I     Hae I    Fok I  Mnl I          Mae I              Spe I
  |   | |      |        | | |     |    | | |    |        |        |        Bsp1286 I
  |   | |      |        | | |     |    | | |    |        |        |        | |    • |  | |    •
ATAGGCTCCATGGAGATCGGGGTGGCCTCTCTGGATGAGGAGGAACCTCAAAACTAGGGAGGAGAATAACTGGGCACTAG  1040
TATCCGAGGTACCTCTAGCCCCACCGGAGAGACCTACTCCTCCTTGGAGTTTTGATCCCTCCTCTTATTGACCCGTGATC
  |   | | •    |        •  | | |    •   |    |  | |    |        •      |    | •       • |    | |    •
  964         975      983      993    1000          1014            1032
      968     975      984          997      1006          1019            !036
      968     975          986          1002                                  1037
      968
         969
```

```
                                                          Hph I
                                          Mse I           Mnl I
         Mae I            Hinf I          Dra I      Nla III  Mnl I
           |                |               | |         |     | |
TGGCTGTCTGCCTAGTTCATATCAGAATCACTGGGGAGTTTTAAAAGCACCCCATGCCTCACCTCCCAAAAAGGCAGAAAA   1120
ACCGACAGACGGATCAAGTATAGTCTTAGTGACCCCTCAAAATTTTCGTGGGGTACGGAGTGGAGGGTTTTCCGTCTTTT
            • |         •    |        •              | |       •  |    | | • |        •              •        •
             1052           1065             1080          1093       1102
                                           I081                  1097
                                                                 1099


                         Hae III
                         Hae I                            ScrF I
                         ScrF I                           EcoR II
                         EcoR II                          BstN I
                         BstN I              AlwN I
         Nla III         Sec I        Mse I     PflM I        SfaN I            Alu I
           |             || ||          |         | |           |                |
TAAAGCACCCATGCCCAAGCCCCACCCTGGCCTAATTAAATCAGAATACCAGAGCCTGGAGCATCAGCACGCTCCGAAGC   1200
ATTTCGTGGGTACGGGTTCGGGGTGGGACCGGATTAATTTAGTCTTATGGTCTCGGACCTCGTAGTCGTGCGAGGCTTCG
           |           •   || ||•        |       •        | |      |     • |          •           | •
          1130             1145          1156          1169       1181          1198
                          1146                         1170
                          1146                              1175
                          1146                              1175
                           1148                             1175
                           1149

                                                           BstX I
                                                           Hae III
                                                           Gdi II
                                                           Eae I
```

EP 0 496 174 A1

```
                                                                  Msp I
                                                                  Hpa II
                            Mnl I                                 Nae I
                            Dde I                      Tth111 II  Cfr10 I
                            Alu I                      Sau96 I    Hae III      Mae II
                            Pvu II        Fnu4H I      Alu I  Ava II  Sau96 I  Afl III    Nla III
                            NspB II       Bbv I
                            | |  |  |       |            |      |    |    |||||| |    | |       |
TCCCCAGCTGAGGCTGATGAGCAGCAGGAGCGAATACAGCTACAGGTCCAAGCAAAAAGGGCCGGCCAGACACGTGGCTC  1280
AGGGGTCGACTCCGACTACTCGTCGTCCTCGCTTATGTCGATGTCCAGGTTCGTTTTTCCCGGCCGGTCTGTGCACCGAG
     | |  |  |        • |        •        | •      |    |•      |||||| |    | |       |
     1205            1221           1238  1245            1259         1270      1280
     1205            1221                 1245            1260         1272
      1206                                     1249      1261
       1208                                               1261
        1210                                              1262
                                                          1262
                                                           1263
                                                           1263
                                                            1264
                                                             1266
```

12

EP 0 496 174 A1

```
                                                              Sau96 I
                                                              Hae III
                              Sau3A I
                              Mbo  I                          ScrF I
              BstX I          Dpn  I                          EcoR II
   Mse I                Mnl I      Mae III   Alw I   Mnl I    BstN I
     |       |            |          |        ||       |       |   |
ATGCCTTGTTAATCCCAGCACTTTGGGAGGCTGTGACAGGTGGATCACTTGAGGTCAGGAGTTTGAGACCAGCCTGGCCC  1360
TACGGAACAATTAGGGTCGTGAAACCCTCCGACACTGTCCACCTAGTGAACTCCAGTCCTCAAACTCTGGTCGGACCGGG
     |•      |        •    |    •    |      •||     •|      •         •   |   |   •
     1289        1295       1307       1322      1331              1353
                              1313       1323                      1353
                                         1323                      1353
                                         1323                      1356
                                                                   1356
```

```
   Nla III                           Bgl I        Mae III        Alu I      Mnl I
     |                                 |             |             |          |
ACATGGGGAAACCCTGTCTCTACTAAAAATACAAAATTAGCCAGCGTGGCTGTCACGCCTGTAATCCCAGCTATGCGGGA  1440
TGTACCCCTTTGGGACAGAGATGATTTTTATGTTTTAATCGGTCGCACCGACAGTGCGGACATTAGGGTCGATACGCCCT
     |           •           •           •        |        •|        •      |•       |•
     1362                                        1400      1412            1429      1439
```

```
                              Mnl I
                      Sec I
                      ScrF I
                      EcoR II                     Sau3A I
   Mnl I      Hinf I  BstN I      Mnl I           Mbo  I
     |          |       |   |       |             Dpn  I
                                                    |
GGTTGAGGTAGGAGAATCACTTGAACCTGGGAGGCAGAGGTTGCAGTGAGCCAAGATCACGACACTGCACTCCAGCCTGT  1520
CCAACTCCATCCTCTTAGTGAACTTGGACCCTCCGTCTCCAACGTCACTCGGTTCTAGTGCTGTGACGTGAGGTCGGACA
     |      •    |       •     |     •|     |    •            •          •     •          •
     1445       1454           1466      1477          1495
                               1466                   1495
                               1466                   1495
                               1466
                                       1471
```

EP 0 496 174 A1

```
                                   Mbo II
      Ple I                        Mbo II                              Mnl I     Alu I
      Hinf I                       Mbo II                       Nla III         HinD III
       |                            |   |    |                   |   |           ||
GCGACAGAGCGGGACTCCATCTCAAAAAGAAGAAGAAGAAAAGAATAATATGGAGAAAGGGCTGACATGAGGAGAAAAGC  1600
CGCTGTCTCGCCCTGAGGTAGAGTTTTTCTTCTTCTTCTTTTCTTATTATACCTCTTTCCCGACTGTACTCCTCTTTTCG
        •   |        •        | • |    |         •        •          |   | •     || •
          1533              1549                                    1586       1597
          1533                 1552                                   1589       1598      •
                                 1555


        Nla IV
   Mnl I                                             Mnl I                         Fok I
    |          |                                      |                             |
TTGGGGAGGGGGGTTCCTTCCAGTTTAGCGGGCAGAGAGTAGTTCACAGTGGGAGGGTTCACTCTGTTCATTCATTCATC  1680
AACCCCTCCCCCCAAGGAAGGTCAAATCGCCCGTCTCTCATCAAGTGTCACCCTCCCAAGTGAGACAAGTAAGTAAGTAG
   |    •   |          •          •          •       • |       •      •       | •
   1606                                              1653                      1677
       1612


               Mnl I
     Mnl I      Mbo II                      Mae III  Mae I                    Rsa I
      |          |    |                       |        |                       |
CATCTCTCACTCCTTCCCTCCCCCTCTCTTCCTTATTGAGCAAAGTCACTGTGCTAGACACTGCGGATTTGGTACTAAAC  1760
GTAGAGAGTGAGGAAGGGAGGGGGAGAGAAGGAATAACTCGTTTCAGTGACACGATCTGTGACGCCTAAACCATGATTTG
      •        |  • |     |  •              •       |   •   |    •         • |       •
            1697       1707                       1725     1734                1752
              1703

                                          Sau96 I
                                          Nla IV
                                          EcoO109 I
                                          ScrF I
              •        Dde I              EcoR II                        ScrF I
```

EP 0 496 174 A1

```
  Sau96 I                                  HglA I   BstN I                        EcoR II
  Nla IV                        Mnl I            Sec I      Dde I                  BstN I
  Ava II                        Mae III      Bsp1286 I   Hae III                  Mnl I
    |        |'                    |      |        |        ||  ||      |          |   |
AAGCGGAAATGGTCCCTGAGTGGGGAGTAACCCTCTGTGCTCCCCCAGGCCCCTGAGCCTGCGACCTCCTGGCACTGTTG   1840
TTCGCCTTTACCAGGGACTCACCCCTCATTGGGAGACACGAGGGGGTCCGGGGACTCGGACGCTGGAGGACCGTGACAAC
          •|        |     •      |    •  |         |    •   || ||  •  |        •    |   |  •
          1771                  1787        1797          1808          1825
          1771                     1792             1804          1813      1828
          1771                              1797     1805                   1828
               1776                                  1805                   1828
                                                     1805
                                                     1807
                                                     1808
                                                     1808

                     Fok I                 Bsp1286 I                    ScrF I
                     SfaN I                 Nla IV                      EcoR II
                     Fnu4H I                Ban I                       BstN I
  Mae I              Bbv I         Dde I                                Sec I
    |                  |  | |        |    ||                              ||
GGAGTTCTCTAGGGGCAGGCTGAAGGGCAGCATCCGAACTCAGGTGCCCCTGCTCCCAACGCCTTCTCCCCCAGGCTCAC   1920
CCTCAAGAGATCCCCGTCCGACTTCCCGTCGTAGGCTTGAGTCCACGGGGACGAGGGTTGCGGAAGAGGGGGGTCCGAGTG
    |•             •        |  ||      |•  ||      •          •           ||      •
    1849          1867     1879                                           1910
                  1867              1883                                  1911
                     1870           1883                                  1911
                        1871            1884                              1911
                                               Fnu4H I
```

15

AGTGGTACTGGTTCGACTCGCGGGTTCAGTTTCCGAGAGAGTTGTAGTGGTGGGGCCCCGGGCGACGTCCAGCTG
TCACCATGACCAAGCTGAGCGCCCAAGTCAAAGGCTCTCTCAAACATCACCACCCCCGGGGCCCTGCAGGTCGAC

This invention also provides a DNA sequence comprising the promoter of the human villin gene operatively linked to a nucleotide sequence of a structural gene or an oncogene. In specific embodiments of the invention, the structural gene encodes villin or a reporter gene, such as the gene coding for luciferase.

This invention further provides isolated RNA complementary to the promoter sequence of the human villin gene and to the other DNA sequences described herein.

In another embodiment of the invention, a eukaryotic vector is provided. The vector comprises a plasmid or viral vector containing the promoter sequence of the human villin gene.

In addition, this invention provides mammalian cells transformed with the vectors of the invention.

Also, this invention provides transgenic animals containing the promoter of the human villin gene and cell lines cultured from cells of the transgenic animals.

Brief Description of the Drawings

This invention will be more fully described with reference to the drawings in which:

Fig. 1' is a representation of the structure of the isolated 5'-flanking region of the villin gene;

Fig. 2 depicts a partial structure of the human villin gene showing the approximate location of introns and exons;

Fig. 3 depicts the nucleotide sequence of a portion of human villin cDNA, including the location of the intervening sequences (IVS) in the 5'-first 900 base pairs (bp);

Fig. 4'A-B depicts the 5'-region of the nucleotide sequence of DNA encoding a promoter of the human villin gene;

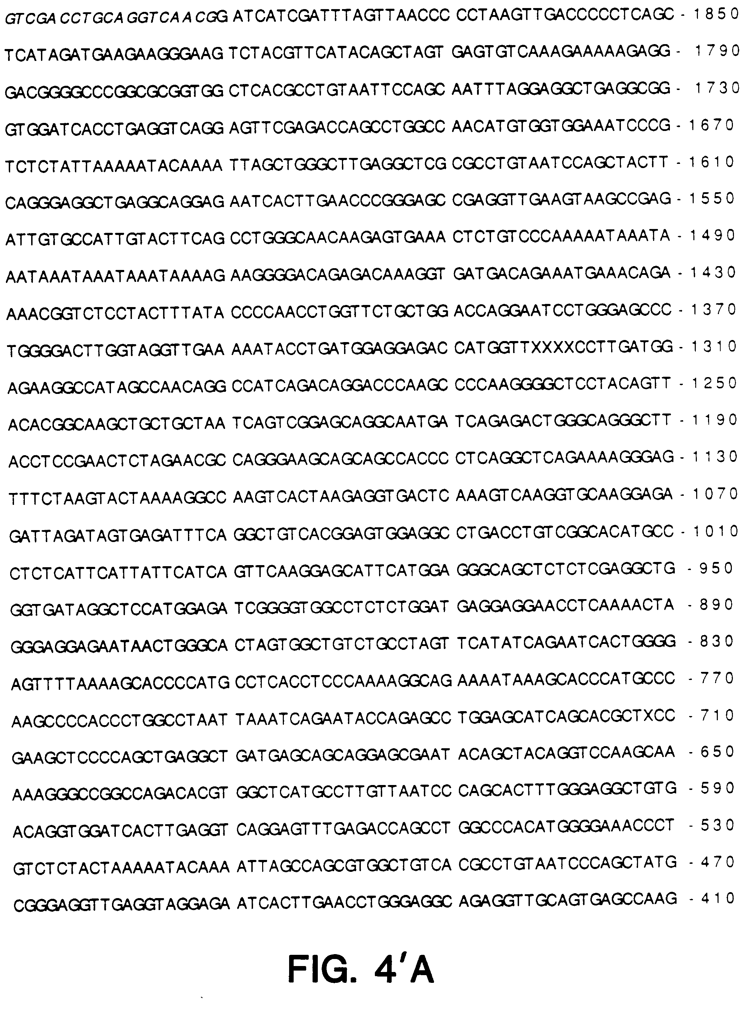

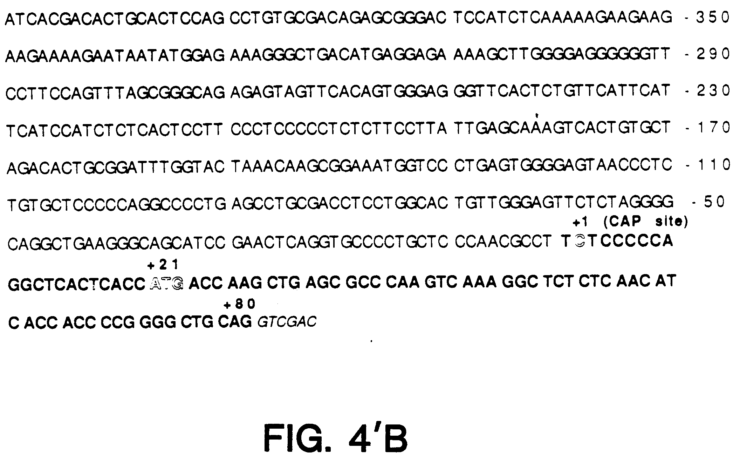

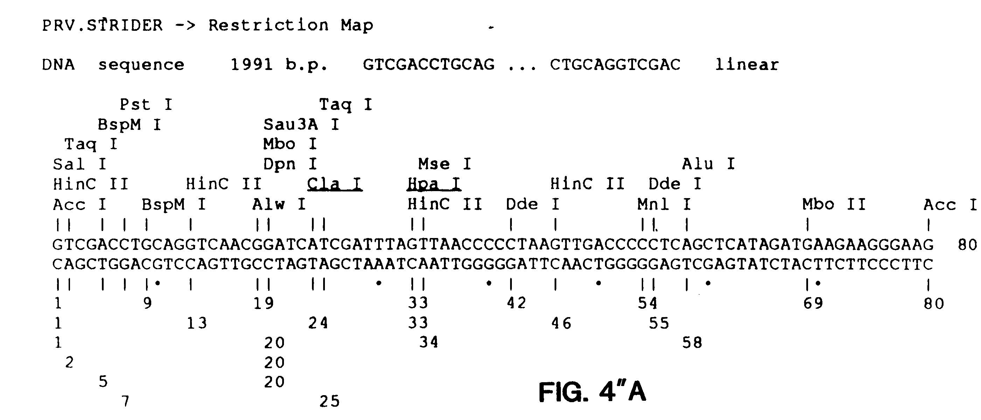

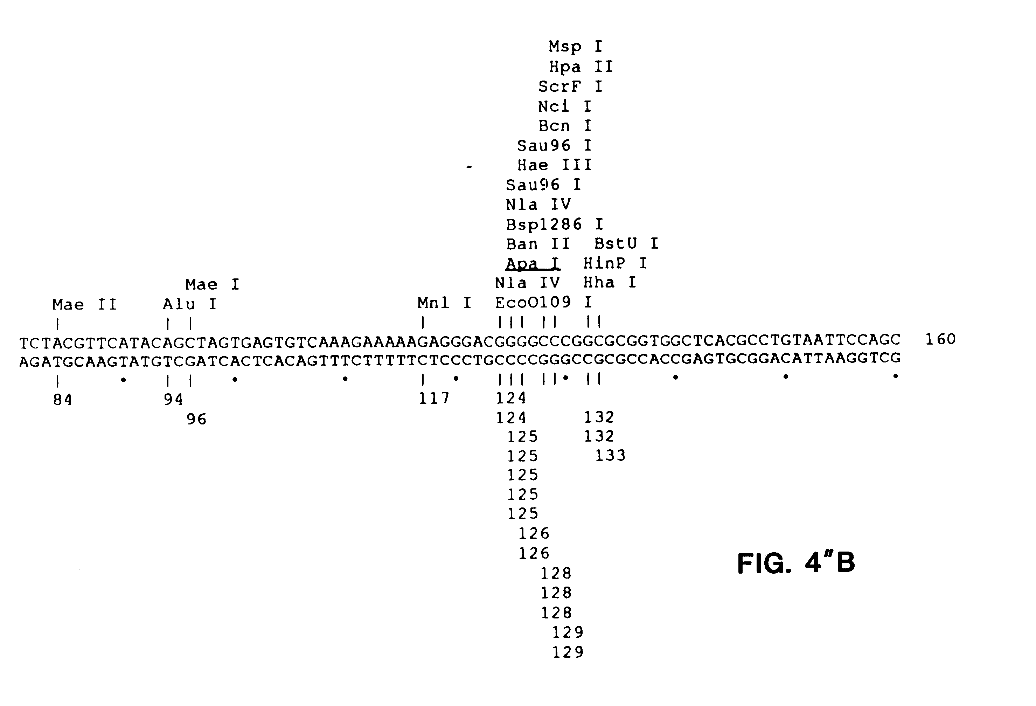

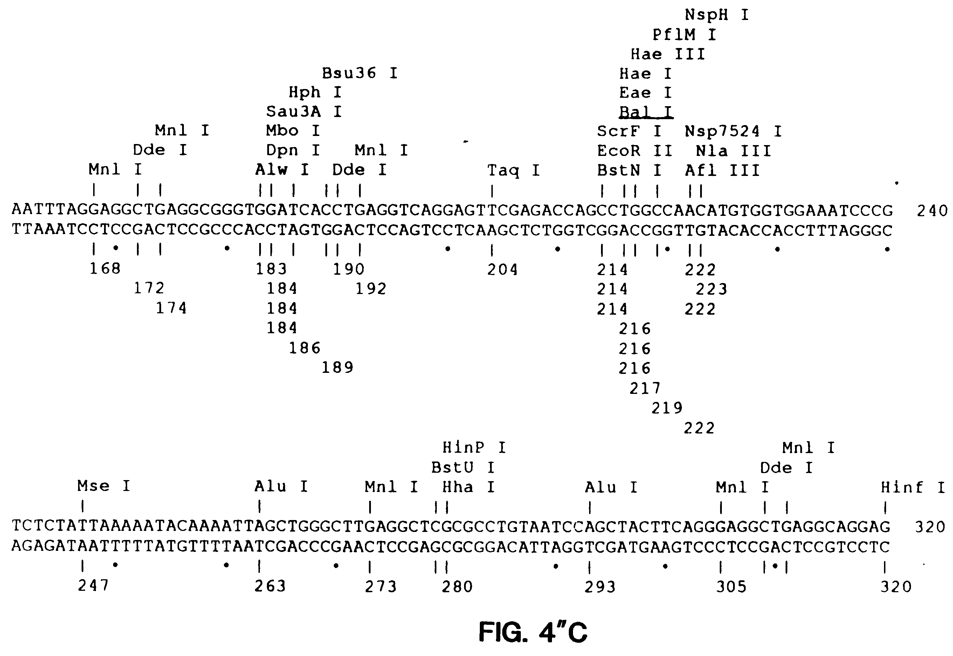

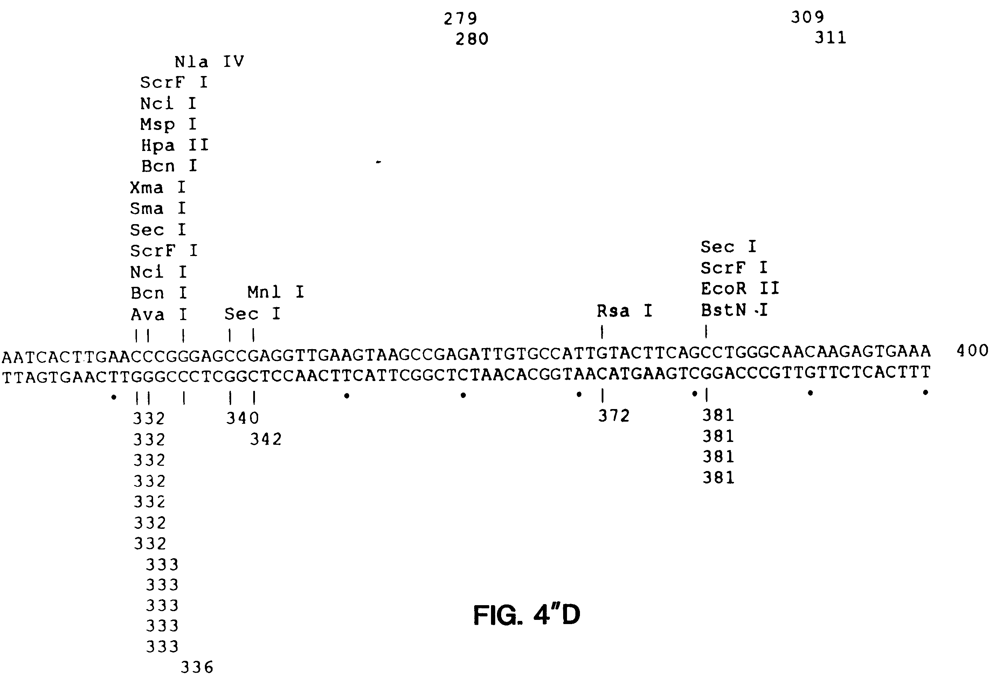

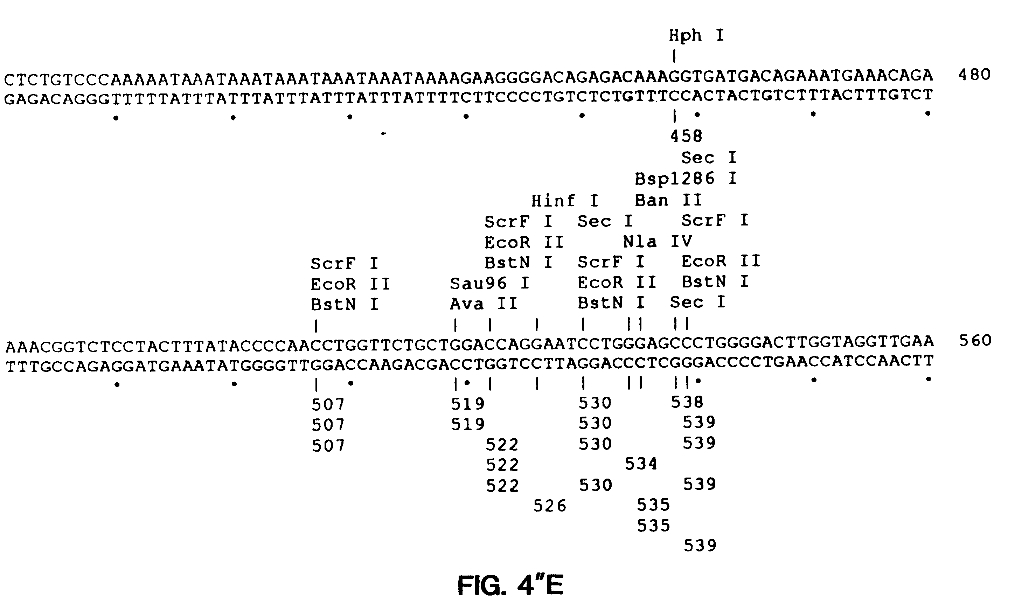

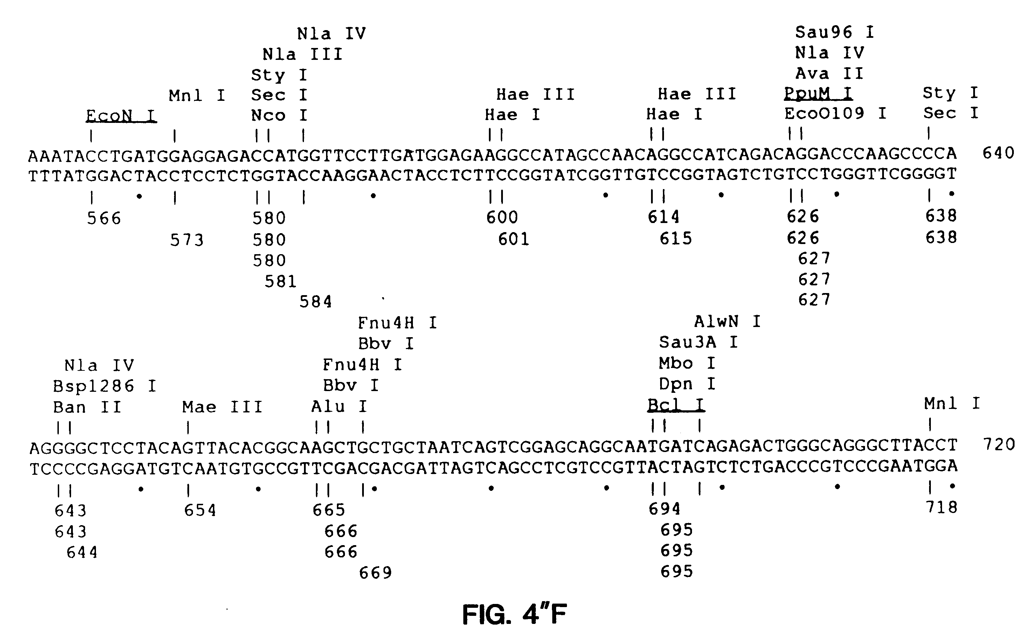

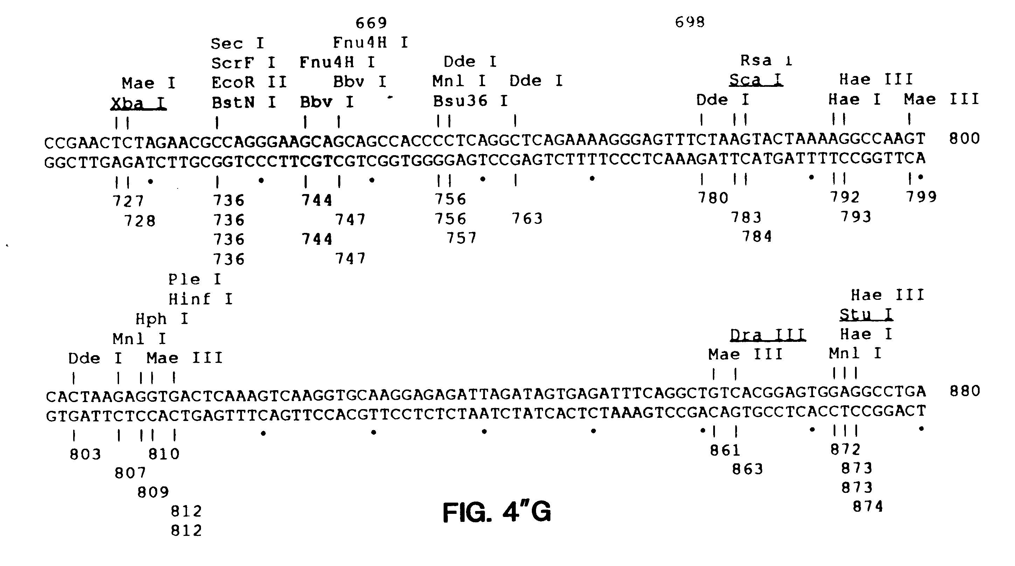

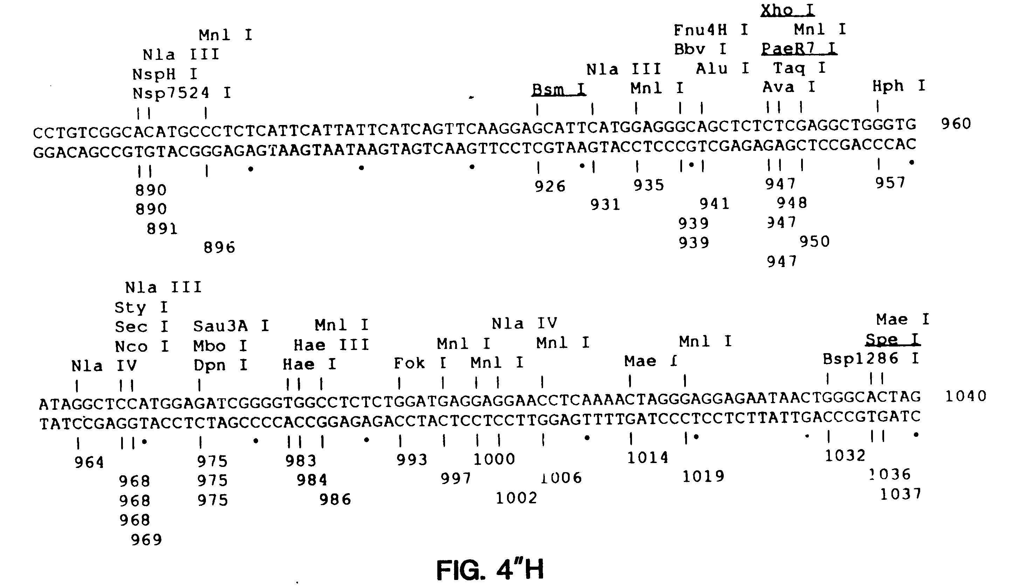

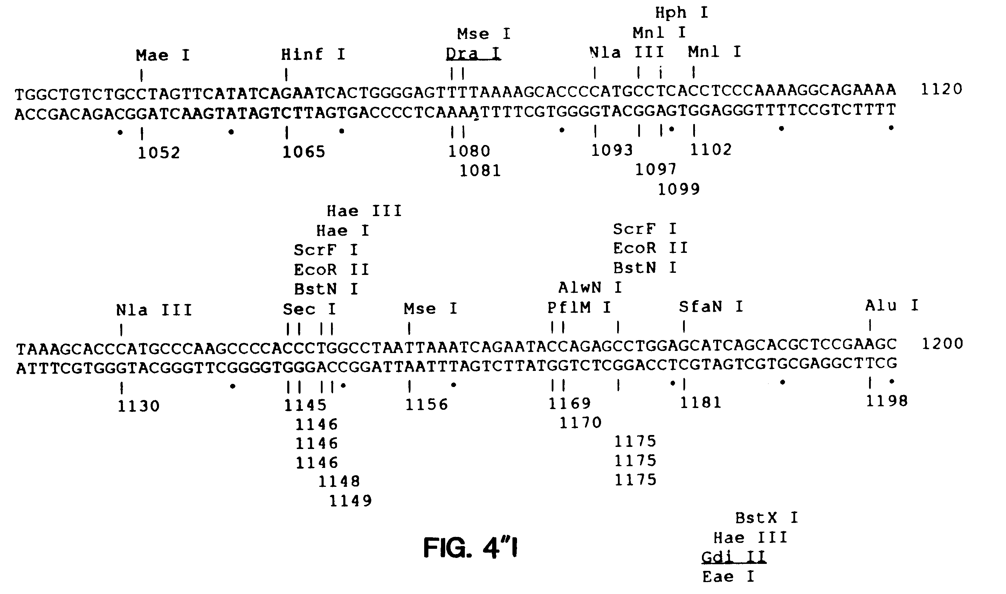

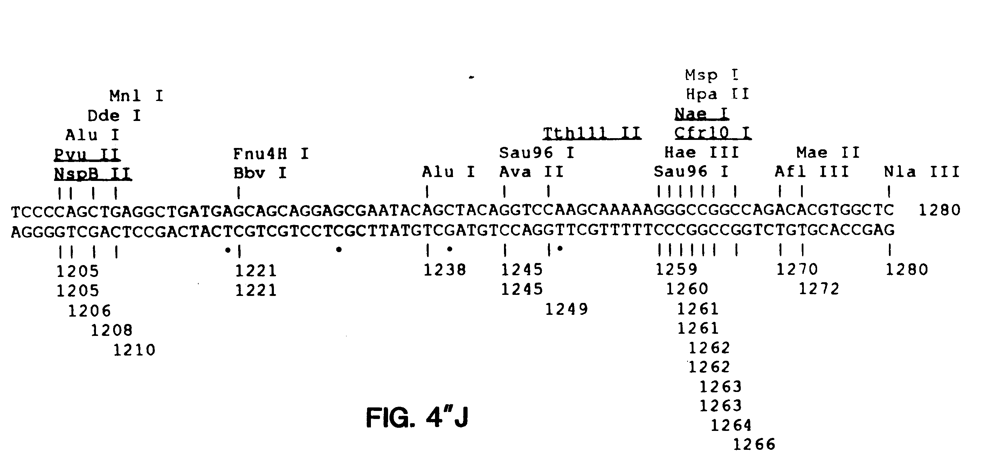

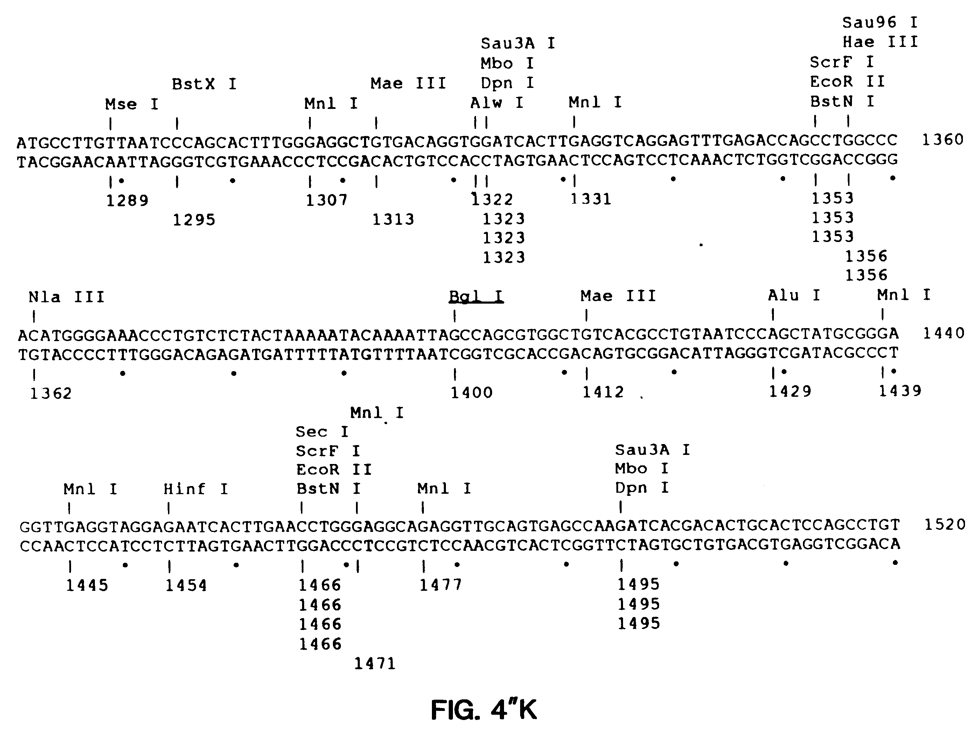

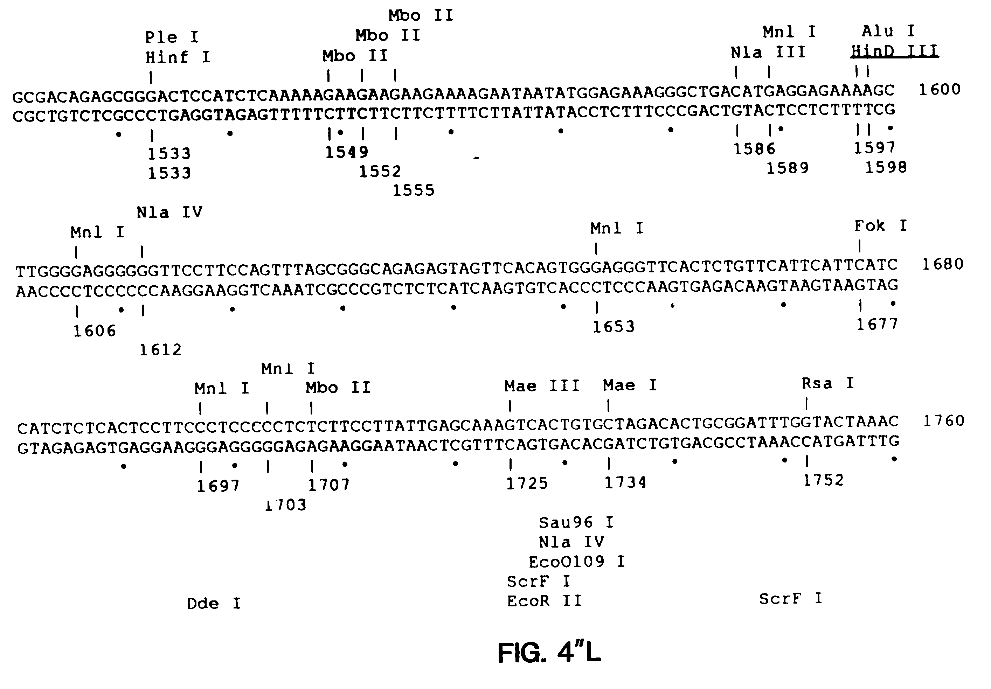

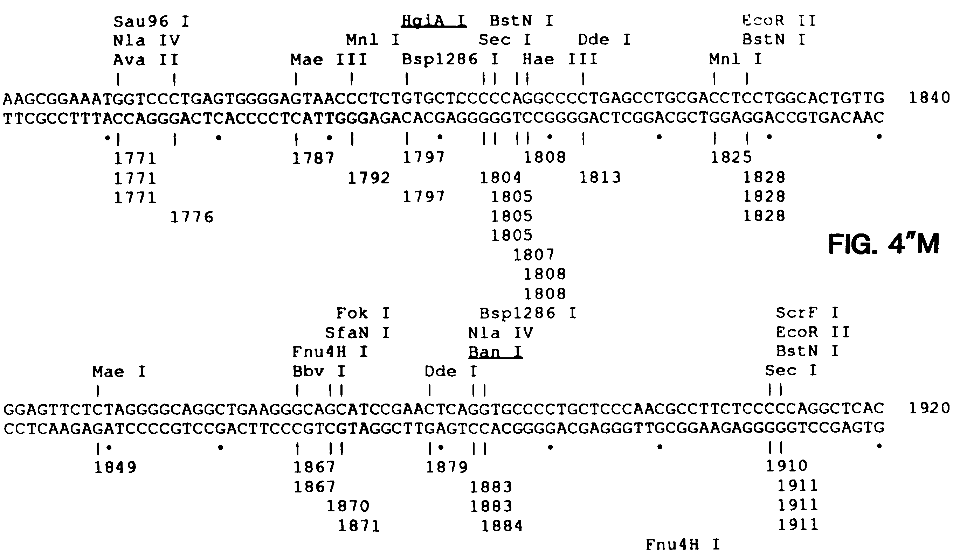

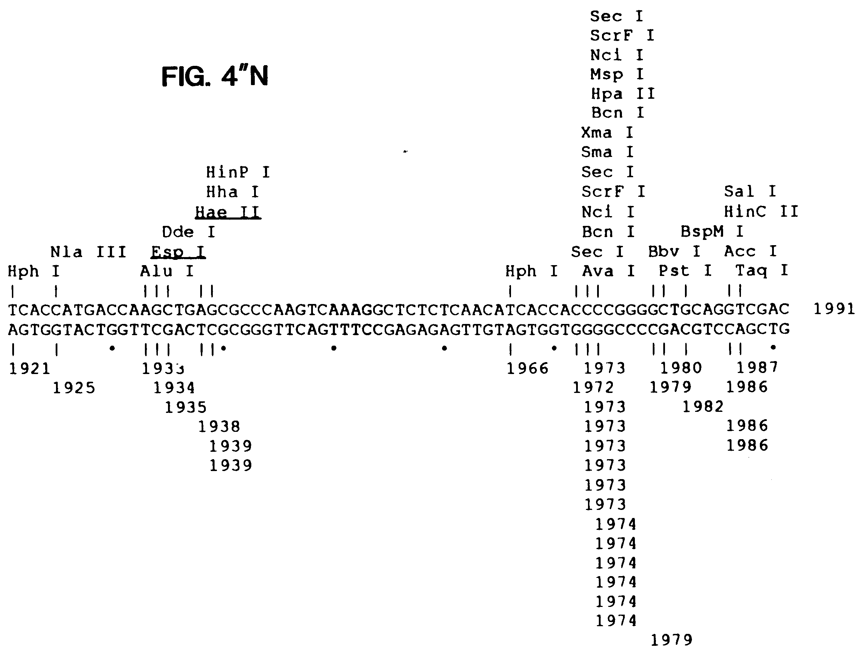

Fig. 4"A-N depicts the nucleotide sequence of the villin gene promoter, including the sequence of complementary strands and restriction endonuclease sites;

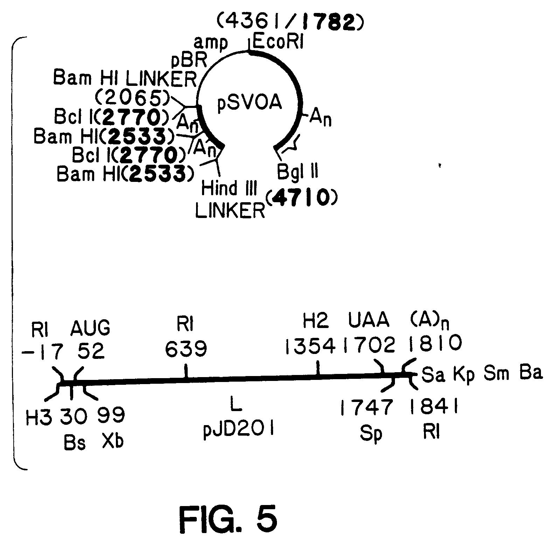

Fig. 5 depicts plasmid vector pSVOA and the luciferase gene employed in one embodiment of the invention;

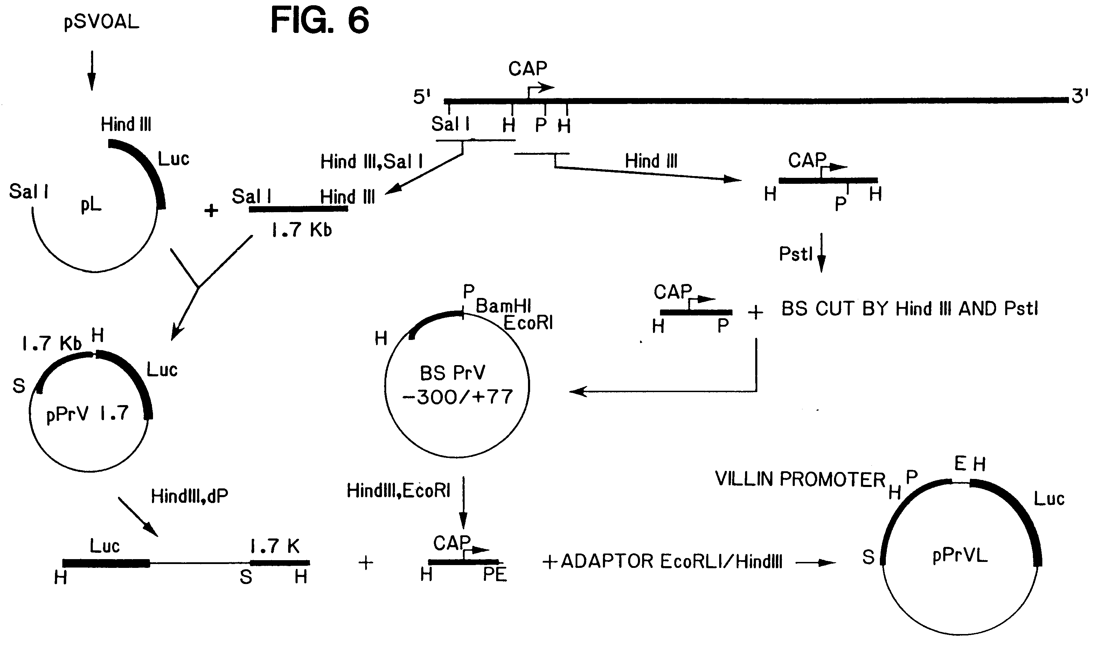

Fig. 6 is a diagrammatic representation of the procedure used to construct plasmid pPrVL of the invention; and

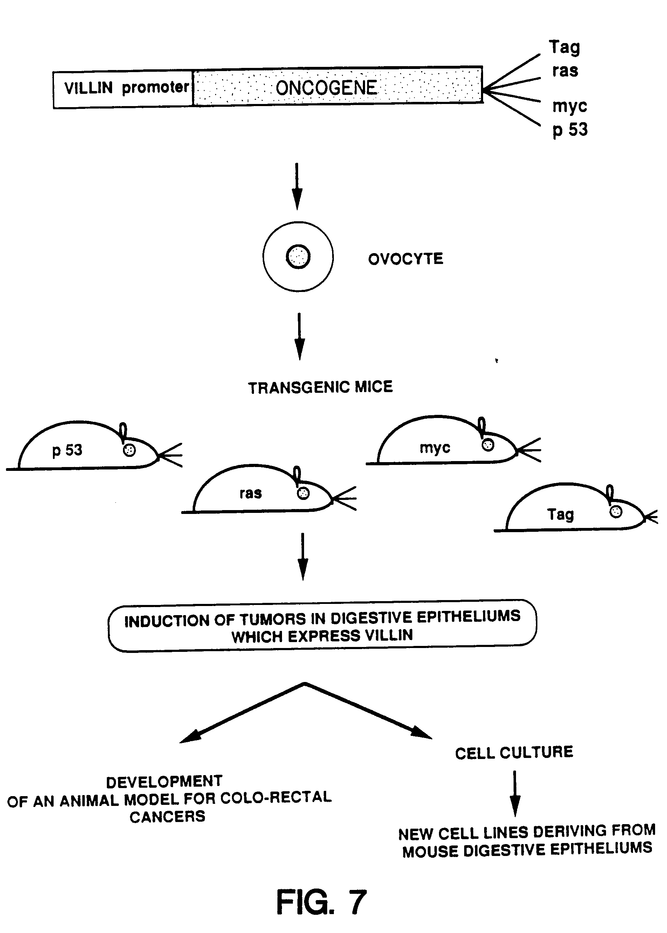

Fig. 7 is a diagrammatic representation of the preparation of an animal model for colorectal cancers and cell cultures derived from animal tumors.

Best Mode for Carrying Out the Invention

1. Gene Promoter Sequence

This invention provides an isolated DNA sequence encoding a promoter of the human villin gene. Villin is a protein with a molecular weight of about 95,000 daltons and is normally present in the villi of the digestive mucosa, more especially in intestinal villi. Villin is able to bind to actin in the presence of calcium ions.

Villin is usually found in brush borders, which are observed at the apex of enterocytes, particularly after they have attained their final stage of differentiation. The enterocytes line the entire intestinal lumen. Micro-villi forming the brush border are assembled at the final stage of differentiation of the enterocyte. Villin is localized in bundles of axial microfilaments of micro-villi. It contributes to the structure of their cytoskeleton. On examining frozen sections by immunofluorescence, it has been observed that villin is present at the apical pole of elongated cells forming columns at the surface of the internal wall of the intestine, and also close to cells of the proximal tubules of the kidney. In both cases, their regions are contiguous with the brush border of the cells concerned.

Villin has not been detected in other types of micro-villi of various other epithelia cells when the micro-villi were not provided with a well-organized brush border. A. Bretscher et al., Exp. Cell Res., **135**:213 (1981); H. Reggio et al., "Membranes in Growth and Development" by A. Liss, New York (1982) pp. 89-105. Furthermore, it has been observed that villin is present in enterocytes from an early stage of their development, well before the organization of the brush border.

Villin can almost always be detected *in vivo* at all stages of the development of tumor cells in the digestive region, at both an early and later stages of tumorogenesis, irrespective of the state of differentiation of the cells concerned. In particularly, villin can be detected in cancers of the colon, one of the most widespread forms of cancer. Villin can also be detected in certain types of renal cancer, but has never been detected in primary tumor cells derived from cancers localized in the tissues of other organs, such as the liver, ovaries, and lungs.

The nucleotide sequence and the amino acid sequence of the COOH-terminal portion of human villin are given below:

```
LYS TRP SER ASN THR LYS SER TYR GLU ASP LEU LYS ALA GLU
AAG TGG AGT AAC ACC AAA TCC TAT GAG GAC CTG AAG GCG GAG

SER GLY ASN SER ARG ASP TRP SER GLN ILE THR ALA GLU VAL
TCT GGC AAC TCT AGG GAC TGG AGC CAG ATC ACT GCT GAG GTC

THR SER PRO LYS VAL ASP VAL PHE ASN ALA ASN SER ASN LEU
ACA AGC CCC AAA GTG GAC GTG TTC AAT GCT AAC AGC AAC CTC

SER SER GLY PRO LEU PRO ILE PHE PRO LEU GLU GLN LEU VAL
AGT TCT GGG CCT CTG CCC ATC TTC CCC CTG GAG CAG CTA GTG

ASN LYS PRO VAL GLU GLU LEU PRO GLU GLY VAL ASP PRO SER
AAC AAG GCT GTA GAG GAG CTC CCC GAG GGT GTG GAC CCC AGC

ARG LYS GLU GLU HIS LEU SER ILE GLU ASP PHE THR GLN ALA
AGG AAG GAG GAA CAC CTG TGC ATT GAA GAT TTC ACT GAG GCC

PHE GLY MET THR PRO ALA ALA PHE SER ALA LEU PRO ARG TRP
TIT GGG ATG ACT CCA GCT GCC TTC TCT GCT CTG CCT CGA TGG

LYS GLN GLN ASN LEU LYS LYS GLU LYS GLY LEU PHE *
AAG CAA CAA AAC CTC AAG AAA GAA AAA GGA CTA ITT TGA GAAG
```

AGTAGCTGTCCTTGTAAAGCAGTACCCTACCCTGATTGTAGGGTCTCATTTTCTCA
CCGATATTAGTCCTACACCAATTGAAGTGAAATTTTGCAGATGTGCCTATGAGCAC
AAACTTCTGTGGCAAATGCCAGTTTTGTTTAATAAATGTACCTATTCCTTCAGAAA
GATGATACCCCAAAAAAAAAAAA

This nucleotide sequence and amino acid sequence identify a domani, which has been termed the headpiece (HP).

The nucleotide sequence coding for the terminal portion of human villin can be isolated by means of the following procedure:

– total mRNA (messenger RNAs) is prepared according to known techniques from a cell line expressing villin;

– a cDNA (complementary DNA) bank is constructed;

– these cDNAs are inserted into a vector capable of expressing the protein coded by the insert;

– by means of the recombinant vectors obtained, a bacterial strain is transformed, then conditions permitting the expression of the desired protein in the bacteria are established; and

– the recombinant clones containing the clone specific for villin is selected by means of antibodies recognizing villin.

These techniques are described in more detail in copending U.S. application Serial No. 287,658, filed December 21, 1988. The sequence of the entire human villin protein has been elucidated by Arpin et al., J. Cell Biol., **107**:1759-1766 (1988) and Pringault et al., EMBO J., **5**:3119-3124 (1986).

A plasmid containing the gene encoding human villin was deposited with the Collection Nationale de Cultures de Microorganismes (C.N.C.M.) of Institut Pasteur in Paris, France on November 13, 1985, under culture collection deposit Accession No. I-497.

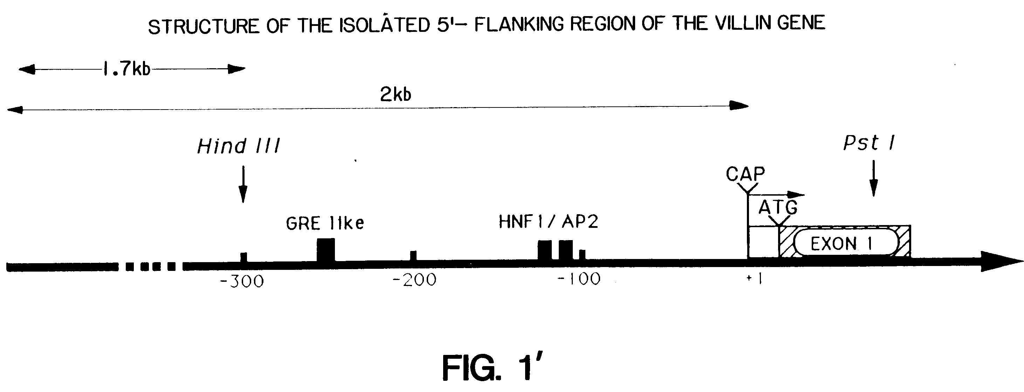

A portion of the operon encoding the human villin gene is diagrammatically shown in Fig. 1'. More particularly, Fig. 1' depicts the structure of an isolated 5'-flanking region of the operon for the human villin gene. Referring to the Figure, a 2 kb region is shown proximate the 5'-end. This region contains the gene promoter of the invention. The CAP site and the ATG start codon of the gene encoding human villin are also shown in the Figure.

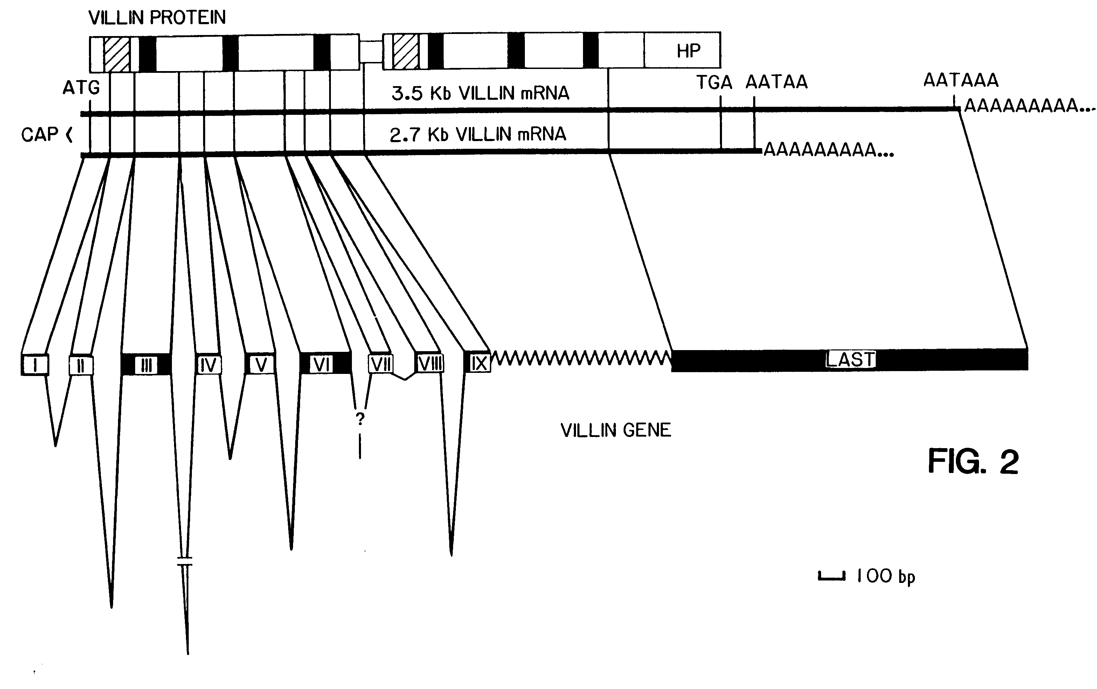

It has been discovered that the operon encoding human villin contains a number of exons and introns. The first exon is shown in Fig. 1' and is identified as "EXON 1". A number of other exons and introns are depicted in Fig. 2, which is a partial structure of an isolated 5'-flanking region of the operon encoding human villin mRNA. Exon 1 in Fig. 1' corresponds to the first exon comprising nucleotides 1-97 in Fig. 2. Introns are identified by triangular regions extending toward the bottom of the Figure. It will be apparent from Fig. 2 that the operon encoding the human villin gene contains a number of intervening sequences.

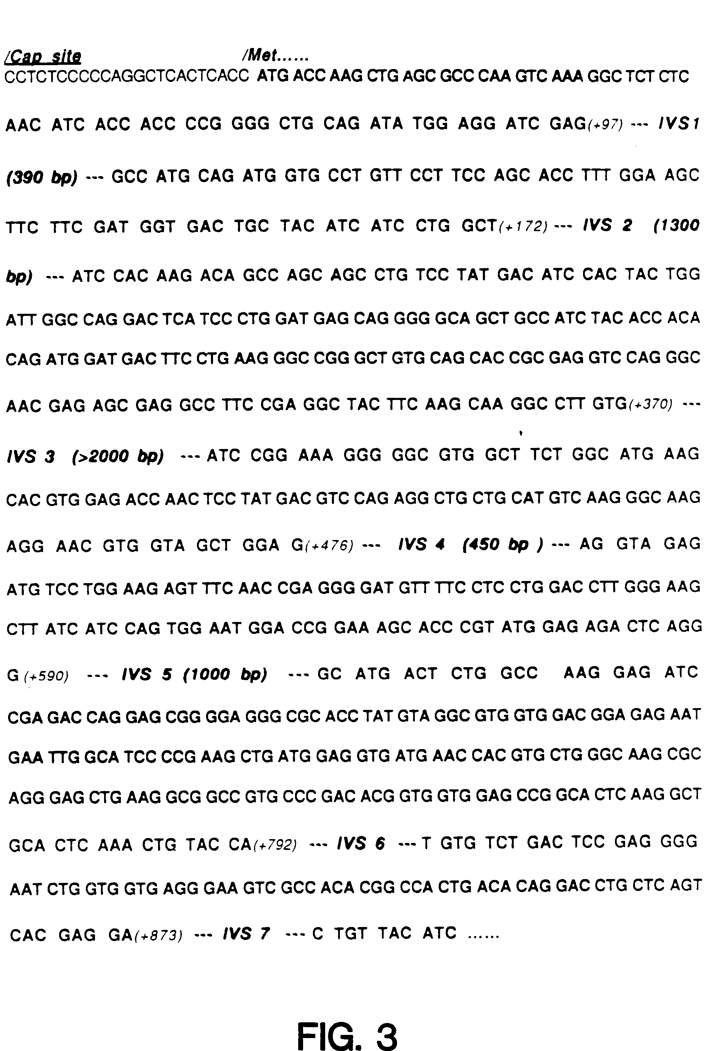

The location of the intervening sequences (IVS) in a 5'-first 900 bp of human villin cDNA is shown in Fig. 3. The IVS are depicted relative to the nucleotide sequence encoding human villin. The sequence shown in Fig. 3 is the coding sequence (exons) of the villin gene. Once again, the CAP site and ATG start codon are shown in the Figure.

The DNA sequence of the invention encodes a promoter of the human villin gene. The DNA sequence is shown in Figs. 4'A-B and 4"A-N. Fig. 4'A-B depicts a portion of the DNA in the 5'-region of the promoter, and includes the CAP site and the ATG codon of the villin gene. Fig. 4"A-N depicts the entire 1991 bp DNA sequence encoding the promoter of the villin gene of the invention. The locations of restriction endonuclease sites in the DNA sequence are indicated in Fig. 4"A-N.

The nucleotide sequence of the promoter of the invention was derived by dideoxynucleotide sequencing. The base sequences of the nucleotides are written in the 5' ----> 3' direction. Each of the letters shown is a conventional designation for the following nucleotides:

A Adenine
G Guanine
T Thymine
C Cytosine.

The DNA sequence encoding the promoter of the human villin gene can be isolated from its natural environment. It is preferred that the DNA sequence encoding the promoter of the human villin gene be in a purified form. For instance, the promoter can be free of human blood-derived proteins, human serum proteins, viral proteins, nucleotide sequences encoding these proteins, human tissue, human tissue components, or combinations of these substances. In addition, it is preferred that the promoter sequence is free of extraneous proteins and lipids, and adventitious microorganisms, such as bacteria and viruses. The essentially purified and isolated DNA sequence encoding the promoter is especially useful for preparing nucleotide probes and expression vectors.

The promoter of the invention can also be prepared by the formation of 3' ----> 5' phosphate linkages between nucleoside units using conventional chemical synthesis techniques. For example, the well-known phosphodiester, phosphotriester, and phosphite triester techniques, as well as known modifications of these approaches, can be employed. Deoxyribonucleotides can be prepared with automatic synthesis machines, such as those based on the phosphoramidite approach. Oligo- and polyribonucleotides can also be obtained with the aid of RNA ligase using conventional techniques.

This invention of course includes variants of the DNA sequence of the invention exhibiting substantially the same properties as the promoter of the invention. By this it is meant that DNA sequences need not be identical to the sequence disclosed herein. Variations can be attributable to single or multiple base substitutions, deletions, or insertions or local mutations involving one or more nucleotides not substantially detracting from the properties of the DNA sequence as a promoter for the human villin gene. It will also be understood that the present invention is intended to encompass fragments of the DNA sequence of the invention in purified form, where the fragments are capable of functioning as promoters for the human villin gene. For example, regulatory elements responsible for tissue specificity of villin can be characterized by a functional test using the Firefly luciferase gene as a reporter gene as described by de Wet et al., *infra*.

The DNA sequence coding for the promoter of the present invention can be amplified in the well known polymerase chain reaction (PCR), which is useful for amplifying all or specific regions of the promoter. See e.g., S. Kwok et al., J. Virol., **61**:1690-1694 (1987); U.S. Patent **4,683,202**; and U.S. Patent 4,683,195. More particularly, DNA primers pairs of known sequence positioned 10-300 base pairs apart that are complementary to the plus and minus strands of the DNA to be amplified can be prepared by well known techniques for the synthesis of oligonucleotides. One end of each primer can be extended and modified to create restriction endonuclease sites when the primer is annealed to the DNA. The PCR reaction mixture can contain the DNA, the DNA primer pairs, four deoxyribonucleoside triphosphates, $MgCl_2$, DNA polymerase, and conventional buffers. The DNA can be amplified for a number of cycles. It is generally possible to increase the sensitivity of detection by using a multiplicity of cycles, each cycle consisting of a short period of denaturation of the DNA at an elevated temperature, cooling of the reaction mixture, and polymerization with the DNA polymerase. Amplified sequences can be detected by the use of a technique termed oligomer restriction (OR). See, R. K. Saiki et al., Bio/Technology **3**:1008-1012 (1985).

The villin gene is strictly regulated during embryonic development of the digestive tract. Villin can be detected in the visceral endoderm, in the primitive gut, in pancreatic and hepatic anlage, as soon as these tissues appear. This expression is then stimulated in differentiated enterocytes, remains low in biliary cells and duct cells, and shut off in hepatocytes and in acinar pancreatic cells. The DNA sequence encoding the promoter of the human villin gene is thus useful to investigate molecular genetics involved in digestive organogenesis. The villin gene promoter strictly regulates the expression of villin in a tissue specific manner, during adult life and during embryonic development.

## 2. Nucleotide Probes Containing the Gene Promoter

The DNA sequence of the invention coding for the promoter of the human villin gene can also be used as a probe for the detection of a nucleotide sequence in a biological material, such as tissue or body fluids. The probe can be labeled with an atom or inorganic radical, most commonly using a radionuclide, but also perhaps with a heavy metal. Radioactive labels including $^{32}P$, $^3H$, $^{14}C$, or the like. Any radioactive label can be employed, which provides for an adequate signal and has sufficient half-life. Other labels include ligands that can serve as a specific binding member to a labeled antibody, fluorescers, chemiluminescers, enzymes, antibodies which can serve as a specific binding pair member for a labeled ligand, and the like. The choice of the label will be governed by the effect of the label on the rate of hybridization and binding of the probe to the DNA or RNA. It will be necessary that the label provide sufficient sensitivity to detect the amount of DNA or RNA available for hybridization.

When the promoter sequence of the invention is used as a probe for hybridizing to a gene, the gene is preferably affixed to a water insoluble solid, porous support, such as nitrocellulose paper. Hybridization can be carried out using labeled polynucleotides of the invention and conventional hybridization reagents. The particular hybridization technique is not essential to the invention.

The amount of labeled probe present in the hybridization solution will vary widely, depending upon the nature of the label, the amount of the labeled probe which can reasonably bind to the support, and the stringency of the hybridization. Generally, substantial excesses of the probe over stoichiometric will be employed to enhance the rate of binding of the probe to the fixed DNA.

Various degrees of stringency of hybridization can be employed. The more severe the conditions, the greater the complementarity that is required for hybridization between the probe and the polynucleotide for duplex formation. Severity can be controlled by temperature, probe concentration, probe length, ionic strength, time, and the like. Conveniently, the stringency of hybridization is varied by changing the polarity of the reactant solution. Temperatures to be employed can be empirically determined or determined from well known formulas developed for this purpose.

## 3. Nucleotide Sequences Containing the Gene Promoter

This invention also provides the DNA sequence encoding the promoter of the invention linked to nucleic acids, such as nucleic acids comprising a structural gene. The nucleic acid can be obtained from any source, for example, from plasmids, from cloned DNA or RNA, or from natural DNA or RNA from any source, including prokaryotic and eukaryotic organisms. DNA or RNA can be extracted from a biological material, such as biological fluids or tissue, by a variety of techniques including those described by Maniatis et al., Molecular Cloning: A Laboratory Manual, Cold Spring Harbor Laboratory, New York (1982). The nucleic acid will generally be obtained from a bacteria, yeast, virus, or a higher organism, such as a plant or animal. The nucleic acid can be a fraction of a more complex mixture, such as a portion of a gene contained in whole human DNA or a portion of a nucleic acid sequence of a particular microorganism. The nucleic acid can be a fraction of a larger molecule or the nucleic acid can constitute an entire gene or assembly of genes. The DNA can be in a single-stranded or double-stranded form. If the fragment is in single-stranded form, it can be converted to double-stranded form using DNA polymerase according to conventional techniques.

The DNA sequence coding for the promoter of the human villin gene can be linked to a structural gene. As used herein, the term "structural gene" refers to a DNA sequence that encodes through its template or messenger mRNA a sequence of amino acids characteristic of a specific protein or polypeptide. The nucleotide sequence of the invention can function as an expression control sequence, that is, a DNA sequence that controls and regulates expression of the structural gene when operatively linked to the gene. In one embodiment of this invention, the DNA sequence encoding the human villin gene promoter is operatively linked to the structural gene for human villin. In another embodiment of the invention, the DNA sequence of the invention is operatively linked to a structural gene other than the gene encoding human villin.

The nucleotide sequence of the invention coding for the promoter of the human villin gene can be used in transient assay systems based on the use of fusion genes. The fusion gene can consist of the promoter of the invention directing the synthesis of a reporter molecule. The relative amount of the reporter protein synthesized under various conditions is presumed to reflect the ability of the promoter to direct transcription. The transient expression system should be based on the synthesis of an easily assayed reporter protein having minimal or no effect on the physiology of the transfected cell. Examples of suitable reporter systems are chloramphenicol acetyltransferase (CAT) and human growth hormone (hGH). Other systems that are also useful include the luciferase system and ^b-galactosidase, which have been successfully used as reporter genes in eukaryotic cells. Thus, for example, the DNA sequence of the invention can be operatively linked to a reporter gene, such as the gene encoding human growth hormone (hGH) or the gene coding for the enzyme luciferase. The Firefly luciferase gene, its structure, and its expression in mammalian cells are described in de Wet et al., Mol. Cell. Biol. **7**:725-737 (1987).

The DNA sequence encoding the promoter of the human villin gene can also be linked to an oncogene. As used herein, the term "oncogene" means a gene having oncogenic or cancer causing potential. The term as used herein includes cellular oncogenes known as proto-oncogenes, as well as viral oncogenes. When the DNA sequence encoding the promoter for the human villin gene is operatively linked to an oncogene, the resulting polynucleotide is referred to herein as an "activated oncogene sequence."

Previous studies have demonstrated that villin is a marker of digestive epithelium cell lineage. Villin gene expression is strictly regulated during embryogenesis and terminal differentiation. Furthermore, the tissue specific expression of villin is maintained during the carcinogenesis process. Villin can be considered as a marker for colorectal carcinoma and can be used to characterize the colorectal origin of unknown metastasis. As a consequence of these properties, the villin gene is a good candidate to target the expression of oncogenes in the digestive epitheliums of transgenic mice. Oncogenes such as SV40T antigen, whose ability to promote tumors in transgenic mice has been fully demonstrated, K-*ras* or p53, whose mutated forms have been found associated with colorectal cancers in humans, can be placed under the control of the villin promoter of the invention. This makes it possible to obtain transgenic mice developing specific tumors deriving from intestinal epitheliums.

Thus, in a preferred embodiment of the invention, the DNA sequence coding for the promoter of the human villin gene is operatively linked to an oncogene that is associated with tumor cells of the gastrointestinal tract or renal system of the human. Oncogenes associated with colon carcinoma are particularly preferred for use in this invention. Examples of particularly preferred oncogenes are TAG, *ras*, *myc*, and p53 genes. It will be understood, however, that other oncogenes can be linked to the promoter sequence of the invention. For example, the promoter sequence can be linked to oncogenes that have appeared in retroviruses, such as the oncogenes *abl, erb-A, erb-B, ets, fes/fps, fgr, ms, fos, kit, mil/raf, mos, myb, K-ras, rel ros, sis ski, src, yes.* Similarly, the promoter sequence of the invention can be linked to oncogenes that have not appeared in retroviruses, such as the oncogenes *bcl-1, bcl-2, bcr, Blym, dil, int-1,int-2, L-myc, mcf2, mcf3, met, Mlvi-1, Mlvi-2; Mlvi-3 neu N-myc, N-ras, onc-D, pim-1, pvt-1, RMO-int-1, tel-1, Tlym-1, Tlym-2, $T_k$NS-1, tx-1, tx-2, tx-3, tx-4.*

The protein products of many of the oncogenes are protein kinases or protein phosphorylating enzymes. Thus, the activated oncogene sequence can be used to study host expression mechanisms by detecting expression of these products.

## 4. Vectors Containing the Gene Promoter

This invention also provides cloning and expression vectors containing the DNA sequence of the invention coding for the promoter of the human villin gene.

More particularly, the DNA sequence encoding the promoter can be ligated to a vehicle for cloning the sequence. The major steps involved in gene cloning comprise procedures for separating DNA containing the gene of interest from prokaryotes or eukaryotes, cutting the resulting DNA fragment and the DNA from a cloning vehicle in specific sites, mixing the two DNA fragments together, and ligating the fragments to yield a recombinant DNA molecule. The recombinant molecule can then be transferred into a host cell, and the cells allowed to replicate to produce identical cells containing clones of the original DNA sequence.

The vehicle employed in this invention can be any double-stranded DNA molecule capable of transporting the promoter of the invention into a host cell and capable of replicating within the cell. More particularly, the vehicle must contain at least one DNA sequence that can act as the origin of replication in the host cell. In addition, the vehicle must contain two or more sites for insertion of the DNA sequence encoding the promoter of the invention. These sites will ordinarily correspond to restriction enzyme sites at which cohesive ends can be formed, and which are complementary to the cohesive ends on the promoter sequence be ligated to the vehicle. In general, this invention can be carried out with plasmid, bacteriophage, or cosmid vehicles having these characteristics.

The nucleotide sequence of the invention can have cohesive ends compatible with any combination of sites in the vehicle. Alternatively, the sequence can have one or more blunt ends that can be ligated to corresponding blunt ends in the cloning sites of the vehicle. The nucleotide sequence to be ligated can be further processed, if desired, by successive exonuclease deletion, such as with the enzyme Bal 31. In the event that the nucleotide sequence of the invention does not contain a desired combination of cohesive ends, the sequence can be modified by adding a linker, an adaptor, or homopolymer tailing.

It is preferred that plasmids used for cloning nucleotide sequences of the invention carry one or more genes responsible for a useful characteristic, such as selectable marker, displayed by the host cell. In a preferred strategy, plasmids having genes for resistance to two different drugs are chosen. For example, insertion of the DNA sequence into a gene for an antibiotic inactivates the gene and destroys drug resistance. The second drug resistance gene is not affected when cells are transformed with the recombinants, and colonies containing the gene of interest can be selected by resistance to the second drug and susceptibility to the first drug. Preferred antibiotic markers are genes imparting chloramphenicol, ampicillin, or tetracyline resistance to the host cell.

A variety of restriction enzymes can be used to cut the vehicle. The identity of the restriction enzyme will generally depend upon the identity of the ends on the DNA sequence to be ligated and the restriction sites in the vehicle. The restriction enzyme is matched to the restriction sites in the vehicle, which in turn is matched to the ends on the nucleic acid fragment being ligated.

The ligation reaction can be set up using well known techniques and conventional reagents. Ligation is carried out with a DNA ligase that catalyzes the formation of phosphodiester bonds between adjacent 5'-phosphate and the free 3'-hydroxy groups in DNA duplexes. The DNA ligase can be derived from a variety of microorganisms. The preferred DNA ligases are enzymes from *E. coli* and bacteriophage T4. T4 DNA ligase can ligate DNA fragments with blunt or sticky ends, such as those generated by restriction enzyme digestion. *E. coli* DNA ligase can be used to catalyze the formation of phosphodiester bonds between the termini of duplex DNA molecules containing cohesive ends.

The vehicle to which the DNA sequence of the invention has been ligated is useful for cloning the sequence. Cloning can be carried in prokaryotic or eukaryotic cells. The host for replicating the cloning vehicle will of course be one that is compatible with the vehicle and in which the vehicle can replicate. When a plasmid is employed, the plasmid can be derived from bacteria or some other organism or the plasmid can be synthetically prepared. The plasmid can replicate independently of the host cell chromosome or an integrative plasmid (episome) can be employed. The plasmid can make use of the DNA replicative enzymes of the host cell in order to replicate or the plasmid can carry genes that code for the enzymes required for plasmid replication. A number of different plasmids can be employed in practicing this invention. Typical of the plasmids that can be utilized are pBR322, pBR325, ColEl, and RP4. A filamentous, lysogenic phage, such as M13 phage, can be employed for cloning the nucleotide sequences of the invention.

The DNA sequence coding for the promoter of the human villin gene and the other nucleotide sequences of the invention, such as the activated oncogene sequence, can also be ligated to a vehicle to form an expression vector. The vehicle employed in this case is one in which it is possible to express a gene operatively linked to the promoter of the invention in an appropriate host cell. It is preferable to employ a vehicle known for use in expressing genes in mammalian cells. These vehicles include SV40 vectors and eukaryotic expression vectors derived from SV40 vectors. Constructs including the DNA sequence encoding the promoter of the invention associated with the gene coding the large T antigen of SV40 are preferred, because the antigen gives transforming power to permissive cells for the expression of the promoter. Other vectors include those derived from Maloney murine sarcoma virus (MSV), herpes simplex virus, Rous sarcoma virus, the mouse mammary tumor virus (MMTV), vaccinia virus, bovine papillomavirus, human adenovirus, and Maloney murine leukemia virus.

In one embodiment of this invention, the DNA sequence coding for the promoter of the human villin gene was operatively linked to the Firefly luciferase gene, and the resulting nucleotide sequence was ligated to plasmid pSVOA using the techniques described in de Wet et al., *supra.* The plasmid pSVOA is shown in Fig. 5.

Referring to Fig. 5, the structures of the mammalian expression vector pSVOA and the full-length, intronless luciferase gene L are shown. The expression vector pSVOA is derived from pSV2, an SV40 early-region promoter vector. Portions of the vector derived from pBR322 are shown as a thin line, those from SV40 are shown as a heavy line. Coordinates of the endpoints of the pBR322 segments are indicated and correspond to the location of the restriction sites in the published sequence of pBR322. Coordinates indicated in bold type correspond to the locations of the restriction sites in the published sequence of SV40. The vector pSVOA contains the SV40 small-t-antigen intron and an SV40 polyadenylation signal labeled $A_n$. The vector pSVOA contains two copies of the SV40 polyadenylation signal. The full-length, intronless luciferase construct, designated L, is carried as *Hind*III-*Bam*HI fragments in the plasmid vector pUC18. The name of the corresponding plasmid is also indicated below the map. The coordinates of the indicated sites are taken from the sequence shown in Fig. 1 of the de Wet et al. publication, *supra.* Restriction sites are indicated with the following abbreviations: H3, *Hind*III; Ri, *Eco*RI; Bs, *Bsm*I; *Xb*; *Xba*I; H2, *Hinc*II; Sp, *Ssp*I; Sa, *Sac*I; Kp, *Kpn*I; Sm, *Sma*I; Ba, *Bam*HI.

The resulting plasmid derived from pSVOA containing the DNA sequence encoding the promoter of the human villin gene operatively linked to the luciferase gene has been designated pPrVL. The plasmid pPrVL was deposited with the Collection National de Cultures de Microorganismes (C.N.C.M.) of Institut Pasteur in Paris, France on October 26, 1990, under culture collection deposit Accession No. I-1011. The procedure used to prepare the pPRVL construct is diagrammatically shown in Fig. 6.

Referring to Figure 6, details concerning the construction of pPrVL are given. A HindIII-SalI restriction fragment from pSVOAL containing the luciferase (Luc) gene was ligated to a 1.7 kb SalI-HindIII fragment obtained from a human genomic library (lambda EMBL 3). The latter fragment was obtained by screening the library using a cocktail of 5′ to 3′ cDNA probes specific for the human villin gene. This construct is referred to as plasmid pPrV 1.7.

A second plasmid was constructed to clone a HindIII/PstI fragment from the same library containing the CAP site of the villin gene. This plasmid was named PrV -300/+77. A HindIII-EcoRI fragment from this plasmid was then ligated to a HindIII fragment from pPrV 1.7 and an EcoRI-HindIII adaptor. The resulting plasmid (pPrVL) contained the 5′ region of the villin gene (1.4 kb insert), the villin promoter and the luciferase gene.

## 5. Cells Transformed with Vectors Containing the Gene Promoter

The vectors of the invention can be inserted into host organisms using conventional techniques. For example, the vectors can be inserted by transformation, transfection, electroporation, microinjection, or by means of liposomes (lipofection). The preferred method for inserting the vectors into mammalian cells is dependent on cell type; however, calcium phosphate precipitation of DNA has been found to be suitable.

Cloning can be carried in prokaryotic or eukaryotic cells. The host for replicating the cloning vehicle will of course be one that is compatible with the vehicle and in which the vehicle can replicate. Cloning is preferably carried out in bacterial or yeast cells, although cells of fungal, animal, and plant origin can also be employed. The preferred host cells for conducting cloning work are bacterial cells, such as *E. coli*, as well as species of *streptomyces, Bacillus,* and *Pseudomonas.* The use of *E. coli* cells is particularly preferred because most cloning vehicles, such as bacterial plasmids and bacteriophages, replicate in these cells.

In a preferred embodiment of this invention, an expression vector containing the DNA sequence encoding the promoter of the invention operatively linked to a structural gene is inserted into a mammalian cell using conventional techniques. For example, the vector can be transfected into a mammalian cell by the calcium phosphate precipitation method or the DEAE-dextran method. A preferred method based on calcium phosphate precipiation is disclosed in Chen and Olsyama, Mol. Cell. Biol., 7:2745-2752 (1987).

Suitable host cells include cells that express villin and cells that do not express villin. Host cells that are preferred for this purpose include human intestinal cell lines derived from colic adenocarcinoma, such as HT29 or CaCO2 cells; human epithelial cell lines, such as C33 cells; and human hepatoma cell lines, such as HepG2 cells; cell lines originating from the renal, proximal tubule of a pig, such as LLCPK1; and cells that do not express villin, such as HeLa cells, which originate from an adecarcinoma of the neck of the uterus. After the plasmid has been introduced into the host cell, the structural gene can be expressed using conventional techniques. These cell lines can be used with recombinant vectors containing the Firefly luciferase gene under the control of the DNA sequence encoding the promoter of the invention in order to isolate regions of the promoter that can be employed for the specific expression of the villin gene in a limited number of tissues. Cell lines have been transfected with plasmid pPRVL of the invention. This plasmid contains the Firefly luciferase reporter gene.

## 6. Transgenic, Non-Human Mammals

Normal mice do not develop intestinal tumors spontaneously. It is possible, however, to induce adenocarcinomas from small and large intestines by using chemical agents. The histology of these tumors resembles that of corresponding human tumors, and they have been used to study external factors involved in the carcinogenesis process. The usefulness of this model is, however, limited for two main reasons. First of all, the model reproduces only some aspects of human carcinogenesis. For instance, metastasis are not observed, especially in the liver. Secondly, tumors have to be induced chemically in each mouse to be studied. This reduces the reproducibility of the model. Moreover, tumor development is very slow (average 7 months).

Transgenic mouse technology is very suitable to develop animal models of carcinogenesis, since this technology makes it possible to obtain mouse lines that genetically develop specific cancers in a reproducible manner, even those that do not appear spontaneously in this animal.

Accordingly, this invention also provides transgenic animals containing the promoter sequence of the invention. More particularly, the invention provides a transgenic non-human mammal whose germ cells and somatic cells contain an activated oncogene sequence of the invention introduced into the animal, or an ancestor of the animal (e.g. an F1 animal), at an embryonic stage. Preferably, the non-human mammal is a rodent, such as a mouse. The activated oncogene sequence, when incorporated into the genome of the mammal, increases the probability of the development of neoplasms, particularly malignant tumors, in the animal. In a preferred embodiment of the invention, the neoplasms are analogs of human colon cancer.

There are several methods by which the oncogene sequence of the invention can be introduced into an animal embryo to ensure that it is incorporated into the chromosome of the animal in an activated state. One method is to transfect the embryo with the gene and to select transgenic animals in which the gene has integrated into the chromosome at a locus that results in activation. Other methods involve modifying the oncogene prior to introduction into the embryo. One such method is to transfect the embryo using a vector containing the activated oncogene sequence of the invention. In a preferred embodiment of the invention, the chromosome of the transgenic animal includes an endogenous coding sequence, preferably the p53, *ras, myc,* or Tag gene, which is substantially the same as the oncogene sequence, and transcription of the oncogene sequence is under the control of the promoter sequence of the invention.

Introduction of the activated oncogene sequence of the invention at the fertilized oocyte stage, such as by microinjection, ensures that the oncogene sequence will be present in all of the germ cells and somatic cells of the transgenic animal, such as the mouse. The presence of the activated oncogene sequence in the germ cells of the transgenic animal ensures that all of the descendants of the animal will carry the activated oncogene sequence in all of their germ cells and somatic cells. The activated oncogene sequence of the invention can be introduced at a later embryonic stage, although this might result in the absence of the oncogene from some somatic cells of the parent animal, although descendants of the animal that inherit the oncogene sequence will carry the sequence in all of their germ cells and somatic cells.

The animals of the invention can be used to test a material suspected of being a carcinogen by exposing the animal to the material and determining neoplastic growth as an indicator of carcinogenicity. The animals can also be used to test materials for ability to confer protection against the development of neoplasms. In either case, the animals are treated with the material, and a reduced incidence of neoplasm development compared to untreated animals is detected as an indication of carcinogenicity or protection. The invention also provides methods of exposing treated and untreated animals to carcinogen prior to, after, or simultaneously with treatment with a protective material.

The transgenic animal model of the invention provides an experimental approach to the different early and late steps of carcinogenesis, and in particular for studying oncogene implication and synergy, the apparition of proliferative hyperplasy, which occurs before the development of solid tumors, and the correlation with angiogenesis.

The villin promoter of the invention is the only promoter isolated to date, which is able to drive the expression of an oncogene in the epithelium of the large intestine. Thus, the DNA sequence encoding the promoter of the human villin gene is the only way to obtain transgenic animals developing colorectal carcinogenesis.

7. Cultures of Transgenic Animal Cells

Intestinal cell lines derived from spontaneous colorectal carcinomas are available. These cell lines have been used to establish cell culture models for absorptive enterocytes and goblet cells. However, the establishment of cell lines for PANETH entero-endocrine cells, and M cells has failed. Furthermore, no well defined cell line models for duct cells of the exocrine pancreas and for biliary cells of the liver are available.

The transgenic animals of the invention can be used as a source of cells for cell culture. Cells from the transgenic animals advantageously exhibit desirable properties of both normal and transformed cultured cells. That is, they are normal or nearly normal morphologically and physiologically, but can be cultured for longer, and even indefinite, periods of time.

More particularly, tissues of the transgenic animals of the invention can be cultured in order to obtain stable cell lines, which reproduce the differentiated phenotype of the corresponding epithelium, and which can be analyzed for the presence of the activated oncogene, either by directly analyzing DNA or RNA, or by assaying the tissue for protein expressed by the gene. Cells of tissues carrying the gene can be cultured using standard tissue culture techniques. The cells are useful for studying the functioning of cells from normally difficult to culture tissues. The cells can be used to study terminal differentiation processes. More particularly, cultures of the transgenic animal cells can be used to characterize the epithelial lines that possess specific differentiation criteria, either of the small intestine or the colon, of ductal cells of the pancreas, or of biliary cells of the liver or of the kidney proximal tubule.

Fig. 7 is a diagrammatic representation of the preparation of an animal cell model for colorectal cancers and cell cultures derived from animal tumors.

Referring to Figure 7, details are given concerning the protocol which is followed to obtain the animal and cell culture model for colorectal cancers. A vector containing the villin promoter and an oncogene (preferably p53, *ras, myc* or *Tag*) is transfected into a mouse oocyte. A selection procedure is used to establish which mice carry the desired genes. These animals are maintained as a colony in which the genes can be readily detected in both somatic and germ cells.

Animals carrying the activated oncogene can be then be used to investigate, for example, the induction of tumors in the digestive epithelium where villin is normally expressed. Using these animals one can develop an animal model for colorectal cancers. Alternatively, one can establish cell lines in culture from the digestive epithelium of transgenic animals carrying the villin gene and promoter which might be coupled to *ras* or *myc* to permit the *in vitro* study of colorectal tumor cells.

## Claims

**1/** An isolated DNA sequence corresponding to a promoter of the human villin gene, wherein the nucleotide sequence consists essentially of :

```
PRV.STRIDER -> Restriction Map

DNA    sequence     1991 b.p.    GTCGACCTGCAG ... CTGCAGGTCGAC    linear

              Pst I              Taq I
           BspM I             Sau3A I
         Taq I                Mbo I
         Sal I                Dpn I              Mse I                    Alu I
         HinC II      HinC II      Cla I     Hpa I            HinC II  Dde I
         Acc I    BspM I      Alw I          HinC II   Dde I       Mnl I        Mbo II      Acc I
         | |   | | |     |      | |      | |      | |      |     |       | |  |        |         |
         GTCGACCTGCAGGTCAACGGATCATCGATTTAGTTAACCCCCTAAGTTGACCCCCTCAGCTCATAGATGAAGAAGGGAAG  80
         CAGCTGGACGTCCAGTTGCCTAGTAGCTAAATCAATTGGGGGATTCAACTGGGGGAGTCGAGTATCTACTTCTTCCCTTC
         | |   | | |•  |      | |      | |      •  | |      • |     |     •  | |  | •       |•        |
         1      9           19          33          42        54         69        80
         1          13         24          33          46        55
         1                20          34                       58
         2               20
            5            20
               7            25
```

```
                                            Msp I
                                            Hpa II
                                            ScrF I
                                            Ncl I
                                            Bcn I
                                          Sau96 I
                                          Hae III
                                         Sau96 I
                                         Nla IV
                                        Bsp1286 I
                                        Ban II   BstU I
                                        Apa I    HinP I
                        Mae I           Nla IV   Hha I
      Mae II        Alu I        Mnl I  EcoO109 I
       I             I I          I     III II   II
TCTACGTTCATACAGCTAGTGAGTGTCAAAGAAAAAGAGGGACGGGGCCCGGCGCGGTGGCTCACGCCTGTAATTCCAGC   160
AGATGCAAGTATGTCGATCACTCACAGTTTCTTTTTCTCCCTGCCCCGGGCCGCGCCACCGAGTGCGGACATTAAGGTCG
       I       •     I I      •        •        I   •   III II• II      •         •         •         •
      84            94                         117    124
                      96                              124      132
                                                      125      132
                                                      125         133
                                                      125
                                                      125
                                                      125
                                                       126
                                                       126
                                                        128
                                                        128
                                                        128
                                                         129
                                                         129
```

EP 0 496 174 A1

```
                                                          NspH I
                                                          PflM I
                                                          Hae III
                        Bsu36 I                           Hae I
                         Hph I                             Eae I
                        Sau3A I                            Bal I
          Mnl I         Mbo I                     ScrF I  Nsp7524 I
           Dde I         Dpn I      Mnl I          EcoR II  Nla III
  Mnl  I               Alw I   Dde I       Taq I   BstN I  Afl III
    |     | |           || |   || |          |     | || |   ||
AATTTAGGAGGCTGAGGCGGGTGGATCACCTGAGGTCAGGAGTTCGAGACCAGCCTGGCCAACATGTGGTGGAAATCCCG  240
TTAAATCCTCCGACTCCGCCCACCTAGTGGACTCCAGTCCTCAAGCTCTGGTCGGACCGGTTGTACACCACCTTTAGGGC
    | • | |        •   || |   || |       •     |       •    | || |• ||      •          •
    168    | |         183     190               204        214    222
        172              184      192                        214      223
            174          184                                 214      222
                         184                                 216
                           186                               216
                             189                             216
                                                             217
                                                              219
                                                                222
```

```
                                     HinP I                              Mnl I
                                     BstU I                              Dde I
         Mse I           Alu I       Mnl I  Hha I        Alu I     Mnl I        Hinf I
           |               |           |     ||            |         |    | |      |
TCTCTATTAAAAATACAAAATTAGCTGGGCTTGAGGCTCGCGCCTGTAATCCAGCTACTTCAGGGAGGCTGAGGCAGGAG  320
AGAGATAATTTTTATGTTTTAATCGACCCGAACTCCGAGCGCGGACATTAGGTCGATGAAGTCCCTCCGACTCCGTCCTC
    |   •           •  |       •   |      ||           •   |         •     |   |•|       |
    247             263         273     280            293         305         320
```

```
                                                    279                          309
                                                    280                          311

                        Nla  IV
                       ScrF  I
                       Nçl  I
                       Msp  I
                       Hpa  II
                       Bcn  I
                     Xma  I
                     Sma  I
                     Sec  I
                     ScrF  I                                    Sec  I
                     Ncl  I                                     ScrF  I
                     Bcn  I      Mnl  I                         EcoR  II
                     Ava  I    Sec  I              Rsa  I       BstN ·I
                     | |    |      | |               |           |
AATCACTTGAACCCGGGAGCCGAGGTTGAAGTAAGCCGAGATTGTGCCATTGTACTTCAGCCTGGGCAACAAGAGTGAAA 400
TTAGTGAACTTGGGCCCTCGGCTCCAACTTCATTCGGCTCTAACACGGTAACATGAAGTCGGACCCGTTGTTCTCACTTT
            •   | |    |     | |         •         •         • |         • |      •          •
               332      340                              372       381
               332      342                                        381
               332                                                 381
               332                                                 381
               332
               332
               332
                333
                333
                333
                333
                333
                 336
```

EP 0 496 174 A1

```
                                                              Hph  I
                                                               |
CTCTGTCCCAAAAAATAAATAAATAAATAAATAAATAAAAGAAGGGGGACAGAGACAAAGGTGATGACAGAAATGAAACAGA 480
GAGACAGGGTTTTTATTTATTTATTTATTTATTTTTCTTCCCCTGTCTCTGTTTCCACTACTGTCTTTACTTTGTCT
      •        •        •        •        •        •        •      | •      •        •
                                                                 458
                                                                Sec  I
                                                            Bsp1286 I
                                           Hinf I   Ban  II
                                     ScrF I  Sec I      ScrF I
                                     EcoR II       Nla IV
                   ScrF I            BstN I  ScrF I      EcoR II
                   EcoR II   Sau96 I         EcoR II  BstN I
                   BstN I    Ava II          BstN I   Sec I
                   |          |  |     |      |     | |    | |
AAACGGTCTCCTACTTTATACCCCAACCTGGTTCTGCTGGACCAGGAATCCTGGGAGCCCTGGGGACTTGGTAGGTTGAA 560
TTTGCCAGAGGATGAAATATGGGGTTGGACCAAGACGACCTGGTCCTTAGGACCCTCGGGACCCCTGAACCATCCAACTT
      •        •        |   •        | • |     |      |     | |    | | •    •        •
                      507         519         530       538
                      507         519         530        539
                      507              522    530        539
                                       522              534
                                       522         530   539
                                          526         535
                                                     535
                                                         539
```

30

EP 0 496 174 A1

```
                              Nla IV                                          Sau96 I
                             Nla III                                          Nla IV
                              Sty I                                           Ava II
                  Mnl I       Sec I                  Hae III        Hae III   PpuM I      Sty I
        EcoN I                Nco I                   Hae I         Hae I      EcoO109 I   Sec I
        |           |         | |    |                | |          | |        | |         |
AAATACCTGATGGAGGAGACCATGGTTCCTTGATGGAGAAGGCCATAGCCAACAGGCCATCAGACAGGACCCAAGCCCCA 640
TTTATGGACTACCTCCTCTGGTACCAAGGAACTACCTCTTCCGGTATCGGTTGTCCGGTAGTCTGTCCTGGGTTCGGGGT
        |      •    |         | |    |           •      | |       •   | |     •    | |    • | |  •
        566             580                      600              614          626        638
             573        580                      601              615          626        638
                        580                                                    627
                       581                                                     627
                          584        •                                         627
```

```
                                     Fnu4H I                         AlwN I
                                      Bbv I                          Sau3A I
         Nla IV                      Fnu4H I                         Mbo I
        Bspl286 I                     Bbv I                          Dpn I
        Ban II          Mae III       Alu I                          Bcl I                Mnl I
        | |             |             | |    |                       | |    |            |
AGGGGCTCCTACAGTTACACGGCAAGCTGCTGCTAATCAGTCGGAGCAGGCAATGATCAGAGACTGGGCAGGGCTTACCT 720
TCCCCGAGGATGTCAATGTGCCGTTCGACGACGATTAGTCAGCCTCGTCCGTTACTAGTCTCTGACCCGTCCCGAATGGA
        | |       •.    |         •   | |    |•           •               •     | |    | •        •      | •
        643            654            665                                    694               718
        643                           666                                    695
         644                          666                                    695
                                        669                                  695
```

31

EP 0 496 174 A1

```
                              669                                        699

                  Sec  I      Fnu4H I
                  ScrF I      Fnu4H I        Dde I                        Rsa I
        Mae  I    EcoR II       Bbv I     Mnl I  Dde I                    Sca I          Hae III
        Xba I     BstN I        Bbv I     Bsu36 I                  Dde I          Hae I  Mae III
         ||         |          |  |        ||     |                 |  ||          ||      |
    CCGAACTCTAGAACGCCAGGGAAGCAGCAGCCACCCCTCAGGCTCAGAAAAGGGAGTTTCTAAGTACTAAAAGGCCAAGT    800
    GGCTTGAGATCTTGCGGTCCCTTCGTCGTCGGTGGGGAGTCCGAGTCTTTTCCCTCAAAGATTCATGATTTTCCGGTTCA
         ||  •       |    •     |  |    •    ||  •   |         •      |  ||  • ||     |•
         727        736   744        756                           780      792   799
          728         736     747      756      763                  783     793
             736     744            757                               784
             736     747

              Ple I
              Hinf I                                                              Hae III
              Hph I                                                               Stu I
          Mnl I                                             Dra III               Hae I
    Dde  I  Mae III                                         Mae III               Mnl I
     |       |  ||  |                                         |  |                  |||
    CACTAAGAGGTGACTCAAAGTCAAGGTGCAAGGAGAGATTAGATAGTGAGATTTCAGGCTGTCACGGAGTGGAGGCCTGA    880
    GTGATTCTCCACTGAGTTTCAGTTCCACGTTCCTCTCTAATCTATCACTCTAAAGTCCGACAGTGCCTCACCTCCGGACT
     |       |  ||  |        •        •          •          •       •|  |    • |||     •
    803        810                                               861      872
      807                                                          863      873
       809                                                                  873
         812                                                                 874
         812
```

32

Mnl I

NlaIII
NspH I
Nsp7524 I

NlaIII

NlaIII          Fnu4H I       Xho I
Sty I    Mnl I  Bbv I    Mnl I
Sec I    Sau3A I  Alu I  PaeRI
Nco I    Mbo I  Taq I
NlaIV    Dpn I  HaeI  MnlI  Ava I    Mae I
              Hae I  FokI  MnlI  Nla IV       Bsp1286 I
                     MnlI  MnlI       MaeI       SpeI

CCTGTCGGCACATGCCCTCTCTCATTCATTATTCATCAGTTCAAGGAGCATTCATGGAGGGCAGCTCTCTCGAGGGCTGGGTG
GGACAGCCGTGTACGGGAGAGAGTAATAAGTAGTCAAGTTCCTCGTAAGTACCTCCCGTCGAGAGAGCTCCGACCCAC

890  890  891   896   926  931  935  939  939  941  947  947  948  950  957  960
                     941  947

ATAGGCTCCATGGAGATCGGGGTGGCCTCTCTGGATGAGGAGGAACCTCAAAAACTAGGAGGAGAATAACTGGGCACTAG
TATCCGAGGTACCTCTAGCCCCACCGGAGAGACCTACTCCTCCTTGGAGTTTTTGATCCCTCCTCCTTATTGACCCGTGATC

964  968  975  983  993  997  1000  1002  1006  1014  1019  1032  1036  1040
     968  975  984                                               1037
     969  975  986

Hph I

EP 0 496 174 A1

```
                                                            Hph I
                               Mse I                        Mnl I
Mae I          Hinf I          Dra I          Nla III  Mnl I
  I              I              I I             I    I i   I
TGGCTGTCTGCCTAGTTCATATCAGAATCACTGGGGAGTTTTAAAAGCACCCCATGCCTCACCTCCCAAAAGGCAGAAAA   1120
ACCGACAGACGGATCAAGTATAGTCTTAGTGACCCCTCAAAATTTTCGTGGGGTACGGAGTGGAGGGTTTTCCGTCTTTT
       • I      •  I     •         I I      •  I   I I• I    •          •
       1052     1065              1080          1093  1102
                                  1081                1097
                                                     1099
```

```
                          Hae III
                          Hae I                       ScrF I
                          ScrF I                      EcoR II
                          EcoR II                     BstN I
                          BstN I           AlwN I
Nla III                   Sec I       Mse I  PflM I       SfaN I          Alu I
  I                       I I I I       I      I I          I               I
TAAAGCACCCATGCCCAAGCCCCACCCTGGCCTAATTAAATCAGAATACCAGAGCCTGGAGCATCAGCACGCTCCGAAGC   1200
ATTTCGTGGGTACGGGTTCGGGGTGGGACCGGATTAATTTAGTCTTATGGTCTCGGACCTCGTAGTCGTGCGAGGCTTCG
  I          •    I I I I•      I    •        I I    I      •I        •       I •
  1130            1145     1156          1169      1181              1198
                  1146                   1170
                  1146                          1175
                  1146                          1175
                      1148                      1175
                      1149
```

```
                                          BstX I
                                          Hae III
                                          Gdi II
                                          Eae I
```

34

EP 0 496 174 A1

```
                                                            Msp I
                    Mnl I                                   Hpa II
                   Dde I                                    Nae I
                  Alu I                          Tth111 II  Cfr10 I
                 Pvu II            Fnu4H I              Sau96 I      Hae III      Mae II
                 NspB II           Bbv I       Alu I    Ava II      Sau96 I   Afl III   Nla III
                 I I  I  I            I            I      I    I       IIIIII I    I I        I
   TCCCCAGCTGAGGCTGATGAGCAGCAGGAGCGAATACAGCTACAGGTCCAAGCAAAAAGGGCCGGCCAGACACGTGGCTC  1280
   AGGGGTCGACTCCGACTACTCGTCGTCCTCGCTTATGTCGATGTCCAGGTTCGTTTTTCCCGGCCGGTCTGTGCACCGAG
      I I  I  I            • I         •          I •     I    I•      IIIIII I    I I        I
      1205                 1221                   1238    1245           1259      1270       1280
      1205                 1221                           1245           1260         1272
       1206                                                   1249       1261
        1208                                                             1261
         1210                                                            1262
                                                                        1262
                                                                         1263
                                                                         1263
                                                                          1264
                                                                           1266
```

Mse I   BstX I

ATGCCTTGTTAATCCAGCACTTTGGGAGGCCTGTGACAGGTGGATCACTTGAGGTCAGGAGTTCAGACCAGCCTGGCC
TACGGAACAATTAGGTCGTGAAACCCTCCGACACTGTCCACCTAGTGAACTCCAGTCCTCAAGTCTGGTCGGACCGGG

1289   1295   1307   1313   1322 1323 1323   1331   1353 1353 1353   1356 1356   1360

Mnl I   Mae III   Sau3A I (Mbo I, Dpn I)   Alw I   Mnl I   ScrF I (EcoR II, BstN I)   Sau96 I (Hae III)   ScrF I (EcoR II, Hae III)   Mnl I

Nla III

ACATGGGGGAAACCCTGTCTCTACTAAAAATACAAAATTAGCCAGGCGTGGTGGCGTAATCCCAGCTATGCGGGA
TGTACCCCCTTTGGGACAGAGATGATTTTTATGTTTTAATCGGTCCGCACCGACAGCGGTCGATACGCCCT

1362   1400   1412   1429   1439   1440

Mnl I   Bgl I   Mae III   Alu I   Mnl I

Mnl I   Hinf I   Sec I (ScrF I, EcoR II, BstN I)   Mnl I   Sau3A I (Mbo I, Dpn I)

GGTTGAGGTAGGAGAATCACTTGAACCTGGGAGGCAGAGGTTGCAGTGAGCCAAGATCACGACACTCCAGCCTGT
CCAACTCCATCCTCTTAGTGAACTTGGACCCTCCGTCTCCAACGTCACTCGGTTCTAGTGCTGTGAGGTCGGACA

1445   1454   1466 1466 1466 1466   1471   1477   1495 1495 1495   1520

36

EP 0 496 174 A1

```
                                    Mbo II
            Ple I                   Mbo II
            Hinf I            Mbo II                                    Mnl I      Alu I
              |               |   |   |                          Nla III  HinD III
              |               |   |   |                            |   |      | |
GCGACAGAGCGGGACTCCATCTCAAAAAGAAGAAGAAGAAAAGAATAATATGGAGAAAGGGCTGACATGAGGAGAAAAGC  1600
CGCTGTCTCGCCCTGAGGTAGAGTTTTTCTTCTTCTTCTTTTCTTATTATACCTCTTTCCCGACTGTACTCCTCTTTTCG
          · |        ·      | · |   |        ·            ·           | ·     | | ·
          1533              1549                                     1586    1597
          1533               1552                                         1589    1598   ·
                              1555

            Nla IV
      Mnl I                                           Mnl I                      Fok I
        |         |                                     |                          |
TTGGGGAGGGGGGTTCCTTCCAGTTTAGCGGGCAGAGAGTAGTTCACAGTGGGAGGGTTCACTCTGTTCATTCATTCATC  1680
AACCCCTCCCCCCCAAGGAAGGTCAAATCGCCCGTCTCTCATCAAGTGTCACCCTCCCAAGTGAGACAAGTAAGTAAGTAG
        |    ·  |         ·        ·           ·     · |      ·        ·       | ·
        1606                                        1653                     1677
           1612

                  Mnl I
      Mnl I       Mbo II                   Mae III  Mae I                 Rsa I
        |         |     |                    |        |                     |
CATCTCTCACTCCTTCCCTCCCCCTCTCTTCCTTATTGAGCAAAGTCACTGTGCTAGACACTGCGGATTTGGTACTAAAC  1760
GTAGAGAGTGAGGAAGGGAGGGGGAGAGAAGGAATAACTCGTTTCAGTGACACGATCTGTGACGCCTAAACCATGATTTG
        ·         | · |   | ·               ·   | · |     ·          · |          ·
                1697     1707             1725    1734                 1752
                  1703

                                        Sau96 I
                                        Nla IV
                                        EcoO109 I
                                        ScrF I
            Dde I                       EcoR II                      ScrF I
```

37

EP 0 496 174 A1

```
        Sau96 I                              HgiA I    BstN I                        EcoR II
        Nla IV                         Mnl I           Sec I      Dde I              BstN I
        Ava II              Mae III          Bsp1286 I Hae III                 Mnl I
        I     I'            I         I       I       II  II      I            I   I
AAGCGGAAATGGTCCCTGAGTGGGGAGTAACCCTCTGTGCTCCCCCAGGCCCCTGAGCCTGCGACCTCCTGGCACTGTTG    1840
TTCGCCTTTACCAGGGACTCACCCCTCATTGGGAGACACGAGGGGGTCCGGGGACTCGGACGCTGGAGGACCGTGACAAC     .
        .I    I     .       I   . I       I   .    II  II .  I        .     I   I .          .
        1771              1787      1797         1808              1825
        1771                 1792         1804       1813          1828
        1771                      1797    1805                     1828
              1776                        1805                     1828
                                          1805
                                           1807
                                           1808
                                           1808

                                   Fok I         Bsp1286 I                   ScrF I
                                   SfaN I        Nla IV                      EcoR II
                                Fnu4H I          Ban I                       BstN I
        Mae I                   Bbv I          Dde I                         Sec I
        I                       I  II          I    II                      II
GGAGTTCTCTAGGGGCAGGCTGAAGGGCAGCATCCGAACTCAGGTGCCCCTGCTCCCAACGCCTTCTCCCCCAGGCTCAC    1920
CCTCAAGAGATCCCCGTCCGACTTCCCGTCGTAGGCTTGAGTCCACGGGGACGAGGGTTGCGGAAGAGGGGGTCCGAGTG
        I.                      I  II          I. II       .            .    II          .
        1849                    1867          1879                          1910
                                1867                 1883                   1911
                                   1870             1883                    1911
                                      1871          1884                    1911
                                                Fnu4H I
```

```
Hph I                    Nla III
                         Alu I  Esp I
                         Hae II
                         Hha I  Dde I
                         HinP I

1921      1925  1934 1935 1938 1939
          AGTGGTACTGGTTCGACTCGCGGGTTCAGTTTCCGAGAGAGTTGTAGTGGTCGGGGCCCCGACGTCCAGCTG
          TCACCATGACCAAGCTGAGCGCCCAAGTCAAAGGCTCTCTCAACATCACCACCCCGGGGCCTGCAGGTCGAC

                              Hph I              BspM I
                         Sec I  Ava I  Pst I  Acc I
                         Bcn I  Bbv I         HinC II
                         Ncl I              Sal I  Taq I
                         ScrF I
                         Sec I
                         Sma I
                         Xma I
                         Bcn I
                         Hpa II
                         Msp I
                         Ncl I
                         ScrF I
                         Sec I
```

2/ A DNA sequence comprising the sequence as claimed in claim 1 operatively linked to a nucleotide sequence of an oncogene.

3/ The DNA sequence as claimed in claim 1 or 2, wherein the oncogene is selected from the group consisting of p53, ras, myc and Tag.

4/ An isolated DNA sequence as claimed in claim 1 operatively linked to a structural gene.

5/ An isolated DNA sequence comprising the sequence as claimed in claim 1 or 4 operatively linked to a gene encoding villin.

6/ A DNA sequence comprising the sequence as claimed in claim 1 operatively linked to a gene encoding luciferase.

7/ An isolated RNA sequence complementary to the nucleotide sequence according to anyone of claims 1 to 6.

8/ A vehicle comprising a plasmid a plasmid or viral vector containing the nucleotide sequence as claimed in anyone of claims 1 to 6.

9/ The vehicle as claimed in claim 8, wherein the viral vector is an SV-40 vector.

10/ The vehicle as claimed in claim 8, wherein the plasmid is pSVOAL.

11/ The vehicle as claimed in claim 8 having the identifying characteristics of the vector having culture collection accession number CNCM (Collection Nationale des Microorganismes, Paris, France) I-1011.

12/ The vehicle as claimed in claim 8, wherein the vector is an expression vector.

13/ A transgenic, non-human mammal all of whose germ cells and somatic cells contain a recombinant activated oncogene sequence according to anyone of claims 2 to 5, 5, which has been introduced into the mammal, or an ancestor of the mammal, at an embryonic stage.

14/ The mammal as claimed in claim 13, wherein a chromosome of the mammal includes an endogenous coding sequence substantially the same as a coding sequence of said oncogene sequence.

15/ The mammal as claimed in claim 14, wherein the oncogene sequence has been integrated into a chromosome of the mammal at a site different from the location of the endogenous coding sequence.

16/ The mammal as claimed in anyone of claims 13 to 15, wherein the mammal is a rodent.

17/ The animal as claimed in anyone of claims 13 to 15, wherein the mammal is a mouse.

STRUCTURE OF THE ISOLÂTED 5'− FLANKING REGION OF THE VILLIN GENE

FIG. 1'

FIG. 2

*/Cap site*  */Met......*
CCTCTCCCCCAGGCTCACTCACC ATG ACC AAG CTG AGC GCC CAA GTC AAA GGC TCT CTC

AAC ATC ACC ACC CCG GGG CTG CAG ATA TGG AGG ATC GAG*(+97)* --- *IVS1*

*(390 bp)* --- GCC ATG CAG ATG GTG CCT GTT CCT TCC AGC ACC TTT GGA AGC

TTC TTC GAT GGT GAC TGC TAC ATC ATC CTG GCT*(+172)* --- *IVS 2 (1300*

*bp)* --- ATC CAC AAG ACA GCC AGC AGC CTG TCC TAT GAC ATC CAC TAC TGG

ATT GGC CAG GAC TCA TCC CTG GAT GAG CAG GGG GCA GCT GCC ATC TAC ACC ACA

CAG ATG GAT GAC TTC CTG AAG GGC CGG GCT GTG CAG CAC CGC GAG GTC CAG GGC

AAC GAG AGC GAG GCC TTC CGA GGC TAC TTC AAG CAA GGC CTT GTG*(+370)* ---

*IVS 3 (>2000 bp)* --- ATC CGG AAA GGG GGC GTG GCT TCT GGC ATG AAG

CAC GTG GAG ACC AAC TCC TAT GAC GTC CAG AGG CTG CTG CAT GTC AAG GGC AAG

AGG AAC GTG GTA GCT GGA G*(+476)* --- *IVS 4 (450 bp )* --- AG GTA GAG

ATG TCC TGG AAG AGT TTC AAC CGA GGG GAT GTT TTC CTC CTG GAC CTT GGG AAG

CTT ATC ATC CAG TGG AAT GGA CCG GAA AGC ACC CGT ATG GAG AGA CTC AGG

G *(+590)* --- *IVS 5 (1000 bp)* --- GC ATG ACT CTG GCC  AAG GAG ATC

CGA GAC CAG GAG CGG GGA GGG CGC ACC TAT GTA GGC GTG GTG GAC GGA GAG AAT

GAA TTG GCA TCC CCG AAG CTG ATG GAG GTG ATG AAC CAC GTG CTG GGC AAG CGC

AGG GAG CTG AAG GCG GCC GTG CCC GAC ACG GTG GTG GAG CCG GCA CTC AAG GCT

GCA CTC AAA CTG TAC CA*(+792)* --- *IVS 6* --- T GTG TCT GAC TCC GAG GGG

AAT CTG GTG GTG AGG GAA GTC GCC ACA CGG CCA CTG ACA CAG GAC CTG CTC AGT

CAC GAG GA*(+873)* --- *IVS 7* --- C TGT TAC ATC ......

# FIG. 3

*GTCGA CCTGCA GGTCAA CGG* ATCATCGATTTAGTTAACCC CCTAAGTTGACCCCCTCAGC - 1850

TCATAGATGAAGAAGGGAAG TCTACGTTCATACAGCTAGT GAGTGTCAAAGAAAAAGAGG - 1790

GACGGGGCCCGGCGCGGTGG CTCACGCCTGTAATTCCAGC AATTTAGGAGGCTGAGGCGG - 1730

GTGGATCACCTGAGGTCAGG AGTTCGAGACCAGCCTGGCC AACATGTGGTGGAAATCCCG - 1670

TCTCTATTAAAAATACAAAA TTAGCTGGGCTTGAGGCTCG CGCCTGTAATCCAGCTACTT - 1610

CAGGGAGGCTGAGGCAGGAG AATCACTTGAACCCGGGAGC CGAGGTTGAAGTAAGCCGAG - 1550

ATTGTGCCATTGTACTTCAG CCTGGGCAACAAGAGTGAAA CTCTGTCCCAAAAATAAATA - 1490

AATAAATAAATAAATAAAAG AAGGGGACAGAGACAAAGGT GATGACAGAAATGAAACAGA - 1430

AAACGGTCTCCTACTTTATA CCCCAACCTGGTTCTGCTGG ACCAGGAATCCTGGGAGCCC - 1370

TGGGGACTTGGTAGGTTGAA AAATACCTGATGGAGGAGAC CATGGTTXXXXCCTTGATGG - 1310

AGAAGGCCATAGCCAACAGG CCATCAGACAGGACCCAAGC CCCAAGGGGGCTCCTACAGTT - 1250

ACACGGCAAGCTGCTGCTAA TCAGTCGGAGCAGGCAATGA TCAGAGACTGGGCAGGGCTT - 1190

ACCTCCGAACTCTAGAACGC CAGGGAAGCAGCAGCCACCC CTCAGGCTCAGAAAAGGGAG - 1130

TTTCTAAGTACTAAAAGGCC AAGTCACTAAGAGGTGACTC AAAGTCAAGGTGCAAGGAGA - 1070

GATTAGATAGTGAGATTTCA GGCTGTCACGGAGTGGAGGC CTGACCTGTCGGCACATGCC - 1010

CTCTCATTCATTATTCATCA GTTCAAGGAGCATTCATGGA GGGCAGCTCTCTCGAGGCTG - 950

GGTGATAGGCTCCATGGAGA TCGGGGTGGCCTCTCTGGAT GAGGAGGAACCTCAAAACTA - 890

GGGAGGAGAATAACTGGGCA CTAGTGGCTGTCTGCCTAGT TCATATCAGAATCACTGGGG - 830

AGTTTTAAAAGCACCCCATG CCTCACCTCCCAAAAGGCAG AAAATAAAGCACCCATGCCC - 770

AAGCCCCACCCTGGCCTAAT TAAATCAGAATACCAGAGCC TGGAGCATCAGCACGCTXCC - 710

GAAGCTCCCCAGCTGAGGCT GATGAGCAGCAGGAGCGAAT ACAGCTACAGGTCCAAGCAA - 650

AAAGGGCCGGCCAGACACGT GGCTCATGCCTTGTTAATCC CAGCACTTTGGGAGGCTGTG - 590

ACAGGTGGATCACTTGAGGT CAGGAGTTTGAGACCAGCCT GGCCCACATGGGGAAACCCT - 530

GTCTCTACTAAAAATACAAA ATTAGCCAGCGTGGCTGTCA CGCCTGTAATCCCAGCTATG - 470

CGGGAGGTTGAGGTAGGAGA ATCACTTGAACCTGGGAGGC AGAGGTTGCAGTGAGCCAAG - 410

# FIG. 4'A

ATCACGACACTGCACTCCAG CCTGTGCGACAGAGCGGGAC TCCATCTCAAAAAGAAGAAG  - 350

AAGAAAAGAATAATATGGAG AAAGGGCTGACATGAGGAGA AAAGCTTGGGGAGGGGGGTT  - 290

CCTTCCAGTTTAGCGGGCAG AGAGTAGTTCACAGTGGGAG GGTTCACTCTGTTCATTCAT  - 230

TCATCCATCTCTCACTCCTT CCCTCCCCCTCTCTTCCTTA TTGAGCAAAGTCACTGTGCT  - 170

AGACACTGCGGATTTGGTAC TAAACAAGCGGAAATGGTCC CTGAGTGGGGAGTAACCCTC  - 110

TGTGCTCCCCCAGGCCCCTG AGCCTGCGACCTCCTGGCAC TGTTGGGAGTTCTCTAGGGG  - 50

                                                      +1  (CAP  site)

CAGGCTGAAGGGCAGCATCC GAACTCAGGTGCCCCTGCTC CCAACGCCT T G T CCCCCA

          +21

GGCTCACTCACC ATG ACC AAG CTG AGC GCC CAA GTC AAA GGC TCT CTC AAC AT

               +80

C ACC ACC CCG GGG CTG CAG *GTCGAC*

# FIG. 4′B

PRV.STRIDER -> Restriction Map

DNA sequence    1991 b.p.    GTCGACCTGCAG ... CTGCAGGTCGAC    linear

```
            Pst I                    Taq I
         BspM I                   Sau3A I
       Taq I                      Mbo I
       Sal I                      Dpn I           Mse I                        Alu I
       HinC II        HinC II    Cla I      Hpa I              HinC II  Dde I
       Acc I     BspM I     Alw I           HinC II  Dde I           Mnl I           Mbo II        Acc I
       || | | |    |        || | ||         ||       |    |       |  |  |           |             |
       GTCGACCTGCAGGTCAACGGATCATCGATTTAGTTAACCCCCTAAGTTGACCCCCTCAGCTCATAGATGAAGAAGGGAAG  80
       CAGCTGGACGTCCAGTTGCCTAGTAGCTAAATCAATTGGGGGATTCAACTGGGGGAGTCGAGTATCTACTTCTTCCCTTC
       || | | |•  |         || |   ||    •   ||    • |   |    •   || | |•          |•          |
       1         9          19        33        42        54           69        80
       1              13         24        33        46        55
       1                   20        34                     58
        2                   20
         5                  20
           7                   25
```

## FIG. 4"A

```
                                                Msp I
                                                Hpa II
                                                ScrF I
                                                Nci I
                                                Bcn I
                                              Sau96 I
                                         -      Hae III
                                              Sau96 I
                                              Nla IV
                                              Bsp1286 I
                                              Ban II   BstU I
                                              Apa I    HinP I
                        Mae I                 Nla IV   Hha I
         Mae II         Alu I           Mnl I EcoO109 I
           |              | |             |   ||| ||    ||
TCTACGTTCATACAGCTAGTGAGTGTCAAAGAAAAAGAGGGACGGGGCCCGGCGCGGTGGCTCACGCCTGTAATTCCAGC  160
AGATGCAAGTATGTCGATCACTCACAGTTTCTTTTTCTCCCTGCCCCGGGCCGCGCCACCGAGTGCGGACATTAAGGTCG
   |         •      | |      •           •      |   •   ||| ||• ||        •          •          •          •
  84              94            •                117   124
                   96                                  124      132
                                                       125      132
                                                       125        133
                                                       125
                                                       125
                                                       125
                                                        126
                                                        126
                                                          128
                                                          128
                                                          128
                                                           129
                                                           129
```

## FIG. 4"B

EP 0 496 174 A1

```
                                                                    NspH I
                                                                    PflM I
                                                                    Hae III
                                              Bsu36 I               Hae I
                                              Hph I                  Eae I
                                              Sau3A I                Bal I
                          Mnl I               Mbo I                  ScrF I   Nsp7524 I
                  Dde I                        Dpn I       Mnl I     EcoR II  Nla III
              Mnl I                           Alw I  Dde I           Taq I    BstN I   Afl III

              |     | |                       || |   || |           |        | || |   ||
AATTTAGGAGGCTGAGGCGGGTGGATCACCTGAGGTCAGGAGTTCGAGACCAGCCTGGCCAACATGTGGTGGAAATCCCG  240
TTAAATCCTCCGACTCCGCCCACCTAGTGGACTCCAGTCCTCAAGCTCTGGTCGGACCGGTTGTACACCACCTTTAGGGC
              |  •  | |       •   || |   || |           •   |        •   | || |• ||      •           •
              168               183   190                 204           214   222
                  172               184   192                 214             223
                      174               184                       214           222
                                        184                           216
                                            186                           216
                                                189                           216
                                                                                217
                                                                                  219
                                                                                    222


                                      HinP I                                Mnl I
                                      BstU I                                Dde I
              Mse I             Alu I        Mnl I  Hha I       Alu I        Mnl I        Hinf I
              |                 |            |      ||          |            |     | |    |
TCTCTATTAAAAATACAAAATTAGCTGGGCTTGAGGCTCGCGCCTGTAATCCAGCTACTTCAGGGAGGCTGAGGCAGGAG  320
AGAGATAATTTTTATGTTTTAATCGACCCGAACTCCGAGCGCGGACATTAGGTCGATGAAGTCCCTCCGACTCCGTCCTC
              |  •          • |        •  |      ||          •  |       •    | |•|     |
              247             263           273   280           293          305        320
```

## FIG. 4"C

EP 0 496 174 A1

279
280

309
311

Nla IV
ScrF I
Nci I
Msp I
Hpa II
Bcn I
Xma I
Sma I
Sec I
ScrF I
Nci I

Sec I
ScrF I
EcoR II

Bcn I      Mnl I
Ava I      Sec I                                                    Rsa I     BstN ·I

|  |   |   |  |                                                      |          |
AATCACTTGAACCCGGGAGCCGAGGTTGAAGTAAGCCGAGATTGTGCCATTGTACTTCAGCCTGGGCAACAAGAGTGAAA  400
TTAGTGAACTTGGGCCCTCGGCTCCAACTTCATTCGGCTCTAACACGGTAACATGAAGTCGGACCCGTTGTTCTCACTTT
     •  | |   |   |  |           •           •              • |         • |          •              •
       332       340                                          372       381
       332       342                                                    381
       332                                                              381
       332                                                              381
       332
       332
       332
        333
        333
        333
        333
        333
         336

FIG. 4"D

```
                                                        Hph I
                                                          |
CTCTGTCCCAAAAATAAATAAATAAATAAATAAATAAAAGAAGGGGACAGAGACAAAGGTGATGACAGAAATGAAACAGA   480
GAGACAGGGTTTTTATTTATTTATTTATTTATTTTCTTCCCCTGTCTCTGTTTCCACTACTGTCTTTACTTTGTCT
 •        •         •         •         •         •         | •        •        •
                                                          458
                                                        Sec I
                                                      Bsp1286 I
                                  Hinf I    Ban II
                            ScrF I   Sec I        ScrF I
                            EcoR II       Nla IV
            ScrF I          BstN I   ScrF I      EcoR II
            EcoR II         Sau96 I  EcoR II     BstN I
            BstN I          Ava II   BstN I      Sec I
              |             |  |      |      |   ||    ||
AAACGGTCTCCTACTTTATACCCCAACCTGGTTCTGCTGGACCAGGAATCCTGGGAGCCCTGGGGACTTGGTAGGTTGAA   560
TTTGCCAGAGGATGAAATATGGGGTTGGACCAAGACGACCTGGTCCTTAGGACCCTCGGGACCCCTGAACCATCCAACTT
 •        •         | •      | • |      |      |      ||    ||•       •        •
            507       519         530        538
            507       519         530        539
            507            522     530        539
                          522           534
                          522     530        539
                               526       535
                                         535
                                            539
```

FIG. 4″E

EP 0 496 174 A1

```
                          Nla IV                                                Sau96 I
                          Nla III                                               Nla IV
                          Sty I                                                 Ava II
              Mnl I       Sec I                 Hae III            Hae III       PpuM I           Sty I
     EcoN I               Nco I                 Hae I             Hae I         EcoO109 I         Sec I
       |         |         | |    |              | |              | |           | |               |
     AAATACCTGATGGAGGAGACCATGGTTCCTTGATGGAGAAGGCCATAGCCAACAGGCCATCAGACAGGACCCAAGCCCCA  640
     TTTATGGACTACCTCCTCTGGTACCAAGGAACTACCTCTTCCGGTATCGGTTGTCCGGTAGTCTGTCCTGGGTTCGGGGT
       |    •    |         | |    |         •      | |       •    | |      •    | |  •    | •
       566                 580                    600            614           626            638
              573          580                    601            615           626            638
                          580                                                  627
                         581                                                  627
                            584               •                               627

                                      Fnu4H I                        AlwN I
                                      Bbv I                          Sau3A I
                          Nla IV      Fnu4H I                        Mbo I
              Bsp1286 I              Bbv I                          Dpn I
              Ban II     Mae III     Alu I                         Bcl I                    Mnl I
              | |          |          | |   |                      | |   |                    |
     AGGGGCTCCTACAGTTACACGGCAAGCTGCTGCTAATCAGTCGGAGCAGGCAATGATCAGAGACTGGGCAGGGCTTACCT  720
     TCCCCGAGGATGTCAATGTGCCGTTCGACGACGATTAGTCAGCCTCGTCCGTTACTAGTCTCTGACCCGTCCCGAATGGA
       | |    •    |    •     | |   | •          •         •      | |   | •    •       | •
       643        654        665                          694                        718
       643                   666                          695
        644                  666                          695
                               669                        695
```

FIG. 4"F

EP 0 496 174 A1

669                                              698

Sec I          Fnu4H I
ScrF I      Fnu4H I            Dde I                    Rsa I
Mae I      EcoR II      Bbv I       Mnl I  Dde I        Sca I        Hae III
Xba I       BstN I     Bbv I    -     Bsu36 I          Dde I        Hae I  Mae III
| |          |          |   |        | |        |        |   | |      | |      | •
CCGAACTCTAGAACGCCAGGGAAGCAGCAGCCACCCCTCAGGCTCAGAAAAGGGAGTTTCTAAGTACTAAAAGGCCAAGT  800
GGCTTGAGATCTTGCGGTCCCTTCGTCGTCGGTGGGGAGTCCGAGTCTTTTCCCTCAAAGATTCATGATTTTCCGGTTCA
| | •         |     •     |   |   •     | |  •  |          •        |   | |    • | |    | •
727         736        744           756                 780        792        799
728         736           747        756      763        783        793
            736        744           757                 784
            736           747

Ple I
Hinf I                                                         Hae III
Hph I                                                          Stu I
Mnl I                                          Dra III         Hae I
Dde I   Mae III                                Mae III         Mnl I
|       |  | |  |                              |   |           | | |
CACTAAGAGGTGACTCAAAGTCAAGGTGCAAGGAGAGATTAGATAGTGAGATTTCAGGCTGTCACGGAGTGGAGGCCTGA  880
GTGATTCTCCACTGAGTTTCAGTTCCACGTTCCTCTCTAATCTATCACTCTAAAGTCCGACAGTGCCTCACCTCCGGACT
|       |  | |  |           •           •              •             •  | |      • | | |      •
803      810                                                   861        872
     807                                                       863        873
     809                                                                  873
         812                        FIG. 4″G                              874
         812

EP 0 496 174 A1

```
                                                                    Xho I
                                                     Fnu4H I       Mnl I
          Mnl I                                       Bbv I       PaeR7 I
        Nla III                              Nla III  Alu I       Taq I
        NspH I                               Bsm I   Mnl I        Ava I          Hph I
        Nsp7524 I                              |      |     |   | |     | | |       |
          | |     |                            |      |     |   | |     | | |       |
  CCTGTCGGCACATGCCCTCTCATTCATTATTCATCAGTTCAAGGAGCATTCATGGAGGGCAGCTCTCTCGAGGCTGGGTG  960
  GGACAGCCGTGTACGGGAGAGTAAGTAATAAGTAGTCAAGTTCCTCGTAAGTACCTCCCGTCGAGAGAGCTCCGACCCAC
          | |     |      •          •          •      |    • |     |   | • |     | | |       |   •
          890                                        926         935         947         957
          890                                           931         941         948
          891                                                     939         947
              896                                                 939         950
                                                                            947
```

```
        Nla III
        Sty I
        Sec I    Sau3A I      Mnl I              Nla IV                                Mae I
        Nco I    Mbo I     Hae III     Mnl I    Mnl I         Mnl I                   Spe I
      Nla IV    Dpn I     Hae I    Fok I  Mnl I              Mae I               Bsp1286 I
        |       | |       |       | |   |      |  |  | |     |      |      |           |       | |
  ATAGGCTCCATGGAGATCGGGGTGGCCTCTCTGGATGAGGAGGAACCTCAAAACTAGGGAGGAGAATAACTGGGCACTAG  1040
  TATCCGAGGTACCTCTAGCCCCACCGGAGAGACCTACTCCTCCTTGGAGTTTTGATCCCTCCTCTTATTGACCCGTGATC
        |       | | •      |       •    | | |    •   |     |   | |     |      •      |      | •           • |     | |   •
        964               975     983       993     1000         1014              1032
              968         975     984         997       1006        1019              1036
              968         975         986           1002                              1037
              968
              969
```

## FIG. 4″H

53

```
                                                          Hph I
                                  Mse I                   Mnl I
         Mae I          Hinf I    Dra I          Nla III  Mnl I
           |              |        ||              |   | | |
TGGCTGTCTGCCTAGTTCATATCAGAATCACTGGGGAGTTTTAAAAGCACCCCATGCCTCACCTCCCAAAAGGCAGAAAA  1120
ACCGACAGACGGATCAAGTATAGTCTTAGTGACCCCTCAAAATTTTCGTGGGGTACGGAGTGGAGGGTTTTCCGTCTTTT
         • |        •    |    •    ||        • |   | |•  |        •         •
          1052            1065          1080        1093       1102
                                        1081                 1097
                                                             1099


                      Hae III
                      Hae I                            ScrF I
                      ScrF I                           EcoR II
                      EcoR II                          BstN I
                      BstN I                  AlwN I
         Nla III      Sec I        Mse I      PflM I       SfaN I         Alu I
           |          || ||          |          ||    |      |             |
TAAAGCACCCATGCCCAAGCCCCACCCTGGCCTAATTAAATCAGAATACCAGAGCCTGGAGCATCAGCACGCTCCGAAGC  1200
ATTTCGTGGGTACGGGTTCGGGGTGGGACCGGATTAATTTAGTCTTATGGTCTCGGACCTCGTAGTCGTGCGAGGCTTCG
           |        •    || ||•       |     •     ||    |    •|      •      | •
          1130            1145        1156       1169       1181          1198
                         1146                    1170
                         1146                        1175
                         1146                        1175
                          1148                       1175
                          1149

                                                          BstX I
                                                          Hae III
                       FIG. 4"I                           Gdi II
                                                          Eae I
```

54

EP 0 496 174 A1

```
                                                    Msp I
              Mnl I                                 Hpa II
              Dde I                                 Nae I
              Alu I                    Tth111 II    Cfr10 I
              Pvu II       Fnu4H I         Sau96 I  Hae III    Mae II
              NspB II      Bbv I    Alu I  Ava II   Sau96 I    Afl III   Nla III
              || | |       |        |      |   |    ||||| |    | |       |
TCCCCAGCTGAGGCTGATGAGCAGCAGGAGCGAATACAGCTACAGGTCCAAGCAAAAAGGGCCGGCCAGACACGTGGCTC  1280
AGGGGTCGACTCCGACTACTCGTCGTCCTCGCTTATGTCGATGTCCAGGTTCGTTTTTCCCGGCCGGTCTGTGCACCGAG
              || | |       •|       •      | •    |  |•   ||||| |    | |       |
              1205         1221            1238  1245      1259       1270      1280
              1205         1221                  1245      1260       1272
               1206                                  1249  1261
                1208                                       1261
                 1210                                      1262
                                                          1262
                                                           1263
                                                           1263
                                                            1264
                                                             1266
```

FIG. 4″J

EP 0 496 174 A1

```
                                                               Sau96 I
                                                               Hae III
                                    Sau3A I
                                    Mbo I                       ScrF I
                BstX I               Mae III    Dpn I           EcoR II
        Mse I                Mnl I              Alw I    Mnl I  BstN I
          |       |            |       |         ||        |     |   |
ATGCCTTGTTAATCCCAGCACTTTGGGAGGCTGTGACAGGTGGATCACTTGAGGTCAGGAGTTTGAGACCAGCCTGGCCC  1360
TACGGAACAATTAGGGTCGTGAAACCCTCCGACACTGTCCACCTAGTGAACTCCAGTCCTCAAACTCTGGTCGGACCGGG
          |•    |     •      |  •   |        • ||       •|        •        • |   |   •
         1289        1295        1307        1322    1331              1353
                                     1313       1323                  1353
                                                1323                  1353
                                                1323                  1356
                                                                      1356

   Nla III                                  Bgl I        Mae III          Alu I      Mnl I
    |                                         |              |              |           |
ACATGGGGAAACCCTGTCTCTACTAAAAATACAAAATTAGCCAGCGTGGCTGTCACGCCTGTAATCCCAGCTATGCGGGA  1440
TGTACCCCTTTGGGACAGAGATGATTTTTATGTTTTAATCGGTCGCACCGACAGTGCGGACATTAGGGTCGATACGCCCT
|         •        •         •              |        • |       •        |•          |•
1362                                       1400       1412             1429        1439
```

```
                            Mnl I
                        Sec I
                        ScrF I
                        EcoR II                      Sau3A I
                        BstN I                       Mbo I
   Mnl I      Hinf I       |     |      Mnl I         Dpn I
    |           |          |     |       |              |
GGTTGAGGTAGGAGAATCACTTGAACCTGGGAGGCAGAGGTTGCAGTGAGCCAAGATCACGACACTGCACTCCAGCCTGT  1520
CCAACTCCATCCTCTTAGTGAACTTGGACCCTCCGTCTCCAACGTCACTCGGTTCTAGTGCTGTGACGTGAGGTCGGACA
    |     •    |     •     |    •|      |   •            |     •          •           •
   1445      1454        1466    1477               1495
                        1466                        1495
                        1466                        1495
                        1466
                          1471
```

FIG. 4"K

```
                                    Mbo II
        Ple I                        Mbo II                                    Mnl I       Alu I
        Hinf I                       Mbo II                             Nla III      HinD III
          |                          |   |    |                           |   |          | |
GCGACAGAGCGGGACTCCATCTCAAAAAGAAGAAGAAGAAAAGAATAATATGGAGAAAGGGCTGACATGAGGAGAAAAGC   1600
CGCTGTCTCGCCCTGAGGTAGAGTTTTTCTTCTTCTTCTTTTCTTATTATACCTCTTTCCCGACTGTACTCCTCTTTTCG
        •  |        •      | • |    |       •        •           |   | •      | |  •
          1533              1549                                  1586       1597
          1533                   1552                                  1589       1598
                                 1555

        Nla IV
        Mnl I                                             Mnl I                        Fok I
          |         |                                       |                            |
TTGGGGAGGGGGGTTCCTTCCAGTTTAGCGGGCAGAGAGTAGTTCACAGTGGGAGGGTTCACTCTGTTCATTCATTCATC   1680
AACCCCTCCCCCCAAGGAAGGTCAAATCGCCCGTCTCTCATCAAGTGTCACCCTCCCAAGTGAGACAAGTAAGTAAGTAG
      |   •  |         •        •         •         •   | •          •        |  •
      1606                                              1653                    1677
          1612

                Mnl I
        Mnl I            Mbo II                    Mae III   Mae I                   Rsa I
          |       |        |                         |         |                      |
CATCTCTCACTCCTTCCCTCCCCCTCTCTTCCTTATTGAGCAAAGTCACTGTGCTAGACACTGCGGATTTGGTACTAAAC   1760
GTAGAGAGTGAGGAAGGGAGGGGGAGAGAAGGAATAACTCGTTTCAGTGACACGATCTGTGACGCCTAAACCATGATTTG
        •        |  •  |     |  •              •      |   •    |     •        • |      •
          1697          1707                      1725      1734                  1752
                1703

                                          Sau96 I
                                          Nla IV
                                          EcoO109 I
                                          ScrF I
                Dde I                     EcoR II                              ScrF I
```

## FIG. 4"L

**FIG. 4"M**

```
Sau96 I
Nla IV
Ava II

          1771
          1771
          1771
               1776

AAGCGGAAATGGTCCCTGAGTGGGGGGGAGTAACCTCACCCCTCATTGGGAGACACGAGGGGGTCCCGGGACTCGGACGCCTGGAGGACGTGACAAC
TTCGCCTTTACCAGGGACTCACCCCCTCGTGTTGGAGTGGGGAGTAACCCTCTGTGCTCCCCCAGGGCCCTGAGCCTGCTGGACCTCCTGGCACTGTTG

               1787    Mnl I                    HgiA I              EcoR II
                    1792   1797            Sec I  BstN I            BstN I
                         1797                     Dde I
                    Mae III  Bsp1286 I  Hae III            Mnl I
                              1804 1805 1805
                              1805 1805      1825
                              1807          1828
                              1808 1808     1828
                              1808          1828
                                   1813
                                                                    1840

Mae I        Bbv I     Bsp1286 I
             SfaN I    Nla IV
             Fnu4H I   Dde I
                       Ban I
        1849    1867   1879    ScrF I    ScrF I
             1867    1883      EcoR II   EcoR II
                  1870  1883   BstN I    BstN I
                  1871  1884             Sec I

                              1910       1910
                                   1911  1911
                                   1911  1911

GGAGTTCTCTAGGGGGCAGGCTGAAGGCAGCAATCCGAACTCAGGTGCCCCTGCTCCCAAGCGCCTTCTCCCCAGGCTCAC
CCTCAAGAGATCCCGTCCGACTTCCCGTCGTAGGCTTGAGTCCACGGGGACGAGGGTTGCGGAAGAGGGGGTCCGAGTG

                                                                    Fnu4H I

                                                                    1920
```

EP 0 496 174 A1

# FIG. 4"N

```
                                                              Sec I
                                                              ScrF I
                                                              Nci I
                                                              Msp I
                                                              Hpa II
                                                              Bcn I
                                                              Xma I
                                                              Sma I
                            HinP I                            Sec I
                            Hha I                             ScrF I            Sal I
                            Hae II                            Nci I            HinC II
                          Dde I                               Bcn I     BspM I
          Nla III     Esp I                                  Sec I    Bbv I   Acc I
Hph I                 Alu I                          Hph I    Ava I   Pst I    Taq I
 |      |             | | |    | |                    |      | | |    | | |    | |
TCACCATGACCAAGCTGAGCGCCCAAGTCAAAGGCTCTCTCAACATCACCACCCCGGGGCTGCAGGTCGAC    1991
AGTGGTACTGGTTCGACTCGCGGGTTCAGTTTCCGAGAGAGTTGTAGTGGTGGGGCCCCGACGTCCAGCTG
 |      |      •      | | |    | | •           •           •     |    • | | |    | | |    | |  •
1921           1933                                        1966    1973    1980    1987
      1925           1934                                          1972    1979    1986
            1935                                                   1973           1982
                1938                                               1973                  1986
                  1939                                             1973                  1986
                  1939                                             1973
                                                                  1973
                                                                  1973
                                                                   1974
                                                                   1974
                                                                   1974
                                                                   1974
                                                                   1974
                                                                   1974
                                                                        1979
```

59

FIG. 5

FIG. 6

FIG. 7

**European Patent Office**

# EUROPEAN SEARCH REPORT

Application Number

EP 91 40 2887

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | No relevant documents disclosed<br><br>----- | | C12N15/00<br>C12N15/85<br>C12N15/12<br>A07K67/027 |
| | | | **TECHNICAL FIELDS SEARCHED (Int. Cl.5)**<br><br>C12N<br>C07K |

The present search report has been drawn up for all claims

| Place of search | Date of completion of the search | Examiner |
|---|---|---|
| THE HAGUE | 24 FEBRUARY 1992 | CHAMBONNET F.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)